# EUROPÄISCHE PATENTSCHRIFT

(11) **EP 3 151 774 B1**
(45) Veröffentlichungstag und Bekanntmachung des Hinweises auf die Patenterteilung: **11.07.2018**
(21) Anmeldenummer: 15729763.1
(22) Anmeldetag: 27.05.2015
(51) Int. Cl.: A61B 34/20, A61B 17/15, A61B 90/00, A61B 17/56, A61B 34/10

(54) **MEDIZINISCHES SYSTEM**
MEDICAL SYSTEM
SYSTÈME MÉDICAL

(30) Priorität: 27.05.2014 DE 102014107481
(43) Veröffentlichungstag der Anmeldung: 12.04.2017
(73) Patentinhaber: Aesculap AG, 78532 Tuttlingen (DE)
(72) Erfinder: UTZ, Michael, 78532 Tuttlingen (DE); FIRMBACH, Franz-Peter, 78576 Emmingen-Liptingen (DE)
(74) Vertreter: Hoeger, Stellrecht & Partner Patentanwälte mbB
(86) Internationale Anmeldenummer: PCT/EP2015/061621
(87) Internationale Veröffentlichungsnummer: WO 2015/181188

(56) Entgegenhaltungen:
- EP-A1- 1 958 575
- EP-A1- 2 719 353
- DE-A1- 19 747 427
- US-A1- 2011 257 653

## Beschreibung

Die vorliegende Erfindung betrifft ein medizinisches System, insbesondere zum Implantieren einer Kniegelenkendoprothese, umfassend mindestens eine medizinische Referenzierungseinheit, deren Position im Raum mit einem chirurgischen Navigationssystem detektierbar ist, welche Referenzierungseinheit mindestens ein chirurgisches Markerelement umfasst, das auf einem Trägerelement angeordnet oder ausgebildet und mit einer Nachweiseinrichtung des chirurgischen Navigationssystems detektierbar ist, welche mindestens eine Referenzierungseinheit einen Grundkörper umfasst, der das Trägerelement trägt, wobei der Grundkörper mindestens eine patientenindividuelle, vom Grundkörper weg weisende und von einem Kugeloberflächenausschnitt und von einer ebenen Fläche abweichende Knochenanlagefläche aufweist, welche korrespondierend zu einer Knochenoberfläche des Patienten geformt ist, wobei das System mindestens ein medizinisches Instrument umfasst, welches mit der mindestens einen Referenzierungseinheit lösbar verbindbar ist.

Ein solches System ist beispielsweise aus der DE 10 2010 060 914 A1 bekannt. Für die Positionierung einer oder mehrerer medizinischer Referenzierungseinheiten am Körper eines Patienten ist es erforderlich, zu Beginn einer navigationsgestützten Operation zunächst Landmarken am Körper des Patienten aufzunehmen, beispielsweise durch navigationsgestütztes Palpieren von charakteristischen Punkten. Liegen vor dem Eingriff Bilddaten der Anatomie des Patienten vor, ist es zudem schwierig, die Bilddaten mit navigationsgestützt ermittelten Landmarken beziehungsweise charakteristischen Punkten am Körper des Patienten in Einklang zu bringen. Dies ist insbesondere zu Beginn eines chirurgischen Eingriffs mit einem großen Zeitaufwand und damit mit erheblichen Kosten verbunden.

Unter einem chirurgischen Markerelement im Sinne dieser Patentanmeldung ist insbesondere auch ein definierter Referenzpunkt, beispielsweise in Form einer definierten Vertiefung, deren Position am Trägerelement bekannt ist, zu verstehen. Ein solcher Referenzpunkt kann beispielsweise mit einem navigierten Palpationsinstrument palpiert werden. Dabei kann durch Bestimmung der Position des Palpationsinstruments im Raum dem Referenzpunkt seine Position im Raum zugewiesen werden. Ist an einem Knochen eine weitere Referenzierungseinheit mit mindestens einem, vorzugsweise drei chirurgischen Markerelementen festgelegt, ist dann die Position des definierten Referenzpunktes zur weiteren Referenzierungseinheit bekannt, so dass auch bei einer Bewegung des Knochens mit dem Trägerelement eine Position des definierten Referenzpunktes durch Bestimmung von Lage und Orientierung der weiteren Referenzierungseinheit zu jeder Zeit über das Navigationssystem bestimmbar ist.
Aus der DE 197 47 427 A1 ist ein System zur Knochensegmentnavigation bekannt, das die Merkmale des Oberbegriffs von Anspruch 1 offenbart. Verfahren zum Herstellen von Registrierungsschablonen sind in der EP 2 719 353 A1 beschrieben. In der US 2011/0257653 A1 sind Systeme und Verfahren für patientenbasierte computerunterstützte chirurgische Verfahren offenbart. Die EP 1 958 575 A1 betrifft ein System zum Positionieren oder zum Vorbereiten der Positionierung eines medizinischen Operationsinstruments.
Es ist daher eine Aufgabe der vorliegenden Erfindung, ein System der eingangs beschriebenen Art so zu verbessern, dass dessen Einsatz vereinfacht wird.
Diese Aufgabe wird bei einem medizinischen System der eingangs beschriebenen Art erfindungsgemäß dadurch gelöst, dass das mindestens eine medizinische Instrument eine Sägelehre für einen Femur und/oder eine Tibia umfasst. Die vorgeschlagene Weiterbildung ermöglicht es, die mindestens eine Referenzierungseinheit einfach und sicher in definierter Weise insbesondere an einem Knochen eines Patienten anzulegen und optional an diesem auch zu befestigen. Insbesondere dann, wenn die patientenindividuelle Knochenanlagefläche auf Basis von Bilddaten des Knochens des Patienten hergestellt ist, kann so eine eindeutige Positionierung der mindestens einen Referenzierungseinheit am Knochen des Patienten sicher gestellt werden. Eine zusätzliche Zuordnung und Referenzierung der Position der mindestens einen Referenzierungseinheit am Körper des Patienten zu Beginn des chirurgischen Eingriffs ist in diesem Fall nicht mehr erforderlich. Dieser Schritt wird quasi dadurch vollzogen, dass aufgrund der Ausbildung der patientenindividuellen Knochenanlagefläche und bei Kenntnis der Position des mindestens einen chirurgischen Markerelements relativ zur patientenindividuellen Knochenanlagefläche eine Referenzierung zu Beginn eines chirurgischen Eingriffs redundant wäre. Vorzugsweise hat die patientenindividuelle Knochenanlagefläche eine Größe von mindestens 3 cm². Günstig ist es, wenn diese noch größer ist, beispielsweise mindestens 6 cm². Je größer die patientenindividuelle Knochenanlagefläche, umso sicherer und eindeutiger kann eine genaue Positionierung der mindestens einen Referenzierungseinheit am Körper des Patienten erreicht werden. Das System kann insbesondere zur Implantation künstlicher Gelenke wie künstliche Knie-, Hüft-, oder Schultergelenke eingesetzt werden. Diese Liste ist nicht abschließend. Gemäß der Erfindung ist vorgesehen, dass das medizinische System mindestens ein medizinisches Instrument umfasst, welches mit der mindestens einen Referenzierungseinheit lösbar verbindbar ist. Diese Ausgestaltung ermöglicht es, beispielsweise ein oder mehrere Instrumente mit der Referenzierungseinheit zu verbinden. Ist letztere in definierter Weise am Körper des Patienten festgelegt, kann durch ein definiertes Verbinden des mindestens einen medizinischen Instruments mit der mindestens einen Referenzierungseinheit auch das mindestens eine medizinische Instrument in eindeutig vorgegebener Weise am Körper des Patienten befestigt werden. Günstigerweise umfasst das mindestens eine medizinische Instrument eine Sägelehre für einen Femur und/oder eine Tibia. Mit einer in der beschriebenen Weise in eindeutiger Art und Weise am Knochen eines Patienten festgelegten Sägelehre, und zwar entweder am Femur und/oder an einer Tibia des Patienten, können vor Durchführung eines chirurgischen Eingriffs, beispielsweise zur Vorbereitung von Femur und/oder Tibia für die Implantation eines Teils einer Kniegelenkendoprothese, eindeutige und vordefinierte Sägeschnitte ausgeführt werden, die eine optimale Anpassung des Prothesenteils an den Knochen ermöglichen.

Vorzugsweise sind der Grundkörper und das Trägerelement einstückig ausgebildet oder unlösbar miteinander verbunden. So kann direkt und von einem Operateur oder dessen Hilfspersonal weder absichtlich noch unabsichtlich eine Änderung einer Relativposition zwischen dem Trägerelement und dem Grundkörper und damit auch zwischen dem mindestens einen Markerelement und der patientenindividuelle Knochenanlagefläche verursacht werden. Insgesamt kann so eine Fehleranfälligkeit des Systems minimiert werden.

Ferner kann es vorteilhaft sein, wenn der Grundkörper und das Trägerelement lösbar verbindbar ausgebildet sind. Auf diese Weise kann das Trägerelement mit dem mindestens einen chirurgischen Markerelement mehrfach verwendet werden. Es müssen dann lediglich für jeden Patienten ein oder mehrere Grundkörper mit patientenindividuellen Knochenanlageflächen hergestellt werden, die dann mit dem Trägerelement verbunden werden können.

Günstig ist es, wenn das System eine Kopplungseinrichtung umfasst zum kraft- und/oder formschlüssigen Koppeln des Grundkörpers und des Trägerelements in einer Kopplungsstellung. Mit einer solchen Kopplungseinrichtung können der Grundkörper und das Trägerelement in definierter Weise miteinander verbunden werden. Insbesondere kann die Kopplungseinrichtung ausgebildet sein, um eine eindeutige Kopplung, also nur in einer einzigen Weise, zwischen dem Grundkörper und dem Trägerelement zu ermöglichen.

Auf besonders einfache Weise ausbilden lässt sich die Kopplungseinrichtung, wenn sie erste und zweite Kopplungselemente umfasst, die einerseits am Grundkörper und andererseits am Trägerelement angeordnet oder ausgebildet sind und die in der Kopplungsstellung in Eingriff stehen und in einer Trennstellung außer Eingriff stehen.

Für eine einfache und sichere Kopplung des Grundkörpers und des Trägerelements ist es günstig, wenn die ersten und zweiten Kopplungselemente mindestens eine Kopplungsaufnahme und einen mit dieser zusammenwirkenden, korrespondierenden Kopplungsvorsprung umfassen. Es können auch zwei oder mehr Kopplungsaufnahme oder zwei oder mehr Kopplungsvorsprünge vorgesehen sein, die entweder am Trägerelement oder am Grundkörper oder aber auch an beiden angeordnet oder ausgebildet sind.

Gemäß einer weiteren bevorzugten Ausführungsform der Erfindung kann vorgesehen sein, dass die mindestens eine Referenzierungseinheit mindestens eine Befestigungselementaufnahme für ein Befestigungselement zum Festlegen der Referenzierungseinheit an einem Knochen aufweist. Eine solche Referenzierungseinheit kann einfach und sicher an einem Knochen in definierter Weise festgelegt werden.

Vorzugsweise ist die mindestens eine Befestigungselementaufnahme am Grundkörper und/oder am Trägerelement angeordnet oder ausgebildet. Dies ermöglicht es insbesondere, den Grundkörper oder das Trägerelement getrennt voneinander und wahlweise auch zusammen am Knochen des Patienten mit einem oder mehreren Befestigungselementen festzulegen.

Auf besonders einfache Weise herstellen lässt sich die mindestens eine Befestigungselementaufnahme wenn sie in Form einer Durchbrechung ausgebildet ist. Insbesondere kann sie in Form einer Bohrung ausgebildet sein. Um eine Positionsänderung des Grundkörpers oder des Trägerelements zu vermeiden, ist es günstig, wenn eine die Durchbrechung verlängernde Hülse am Grundkörper und/oder am Trägerelement angeordnet oder ausgebildet ist. In dieser kann ein Knochenpin, eine Knochenschraube oder ein Knochennagel besonders gut geführt werden. Die Hülse kann insbesondere auch eine Richtung für das Einbringen des Knochenbefestigungselements vorgeben und einem Operateur anzeigen.

Auf besonders einfache Weise lassen sich das mindestens eine medizinische Instrument und die mindestens eine Referenzierungseinheit miteinander lösbar verbinden, wenn das medizinische System eine Kupplungseinrichtung zum kraft- und/oder formschlüssigen Kuppeln des mindestens einen medizinischen Instruments und der mindestens einen Referenzierungseinheit in einer Kupplungsstellung umfasst.

Vorteilhaft ist es, wenn die Kupplungseinrichtung erste und zweite Kupplungselemente umfasst, die einerseits an der mindestens einen Referenzierungseinheit und andererseits am mindestens einen medizinischen Instrument angeordnet oder ausgebildet sind und die in der Kupplungsstellung in Eingriff stehen und in einer Lösestellung außer Eingriff stehen. Insbesondere ist es vorteilhaft, wenn mindestens ein Kupplungselement der Kupplungseinrichtung am Grundkörper der mindestens einen Referenzierungseinheit angeordnet oder ausgebildet ist. So kann das mindestens eine medizinische Instrument mit dem Grundkörper der Referenzierungseinheit gekoppelt werden. Wenn insbesondere das Trägerelement vom Grundkörper trennbar ist, kann so optional auch das Trägerelement mit dem mindestens einen Markerelement entfernt werden, der Grundkörper kann dann noch weiter am Körper des Patienten befestigt bleiben.

Auf einfache und sichere Weise lassen sich die mindestens eine Referenzierungseinheit und das mindestens eine medizinische Instrument miteinander verbinden, wenn die ersten und zweiten Kupplungselemente mindestens eine Kupplungsaufnahme und mindestens einen mit dieser zusammenwirkenden, korrespondierenden Kupplungsvorsprung umfassen.

Insbesondere können die Kupplungsaufnahme und der Kupplungsvorsprung ausgebildet sein, um eine kraft- und/oder formschlüssige Verbindung derselben zu ermöglichen.

Um gegebenenfalls das medizinische Instrument unabhängig von der mindestens einen Referenzierungseinheit an einem Knochen eines Patienten festlegen zu können, ist es günstig, wenn das mindestens eine medizinische Instrument mindestens eine Instrumentenbefestigungselementaufnahme für ein Befestigungselement zum Festlegen des mindestens einen medizinischen Instruments an einem Knochen aufweist.

Vorzugsweise ist die mindestens eine Instrumentenbefestigungselementaufnahme in Form einer Durchbrechung ausgebildet. Eine solche lässt sich insbesondere auf einfache Weise herstellen, beispielsweise in Form einer Bohrung.

Gemäß einer weiteren bevorzugten Ausführungsform der Erfindung kann vorgesehen sein, dass das mindestens eine medizinische Instrument einen Anlagekörper umfasst mit mindestens einer patientenindividuellen, vom Anlagekörper weg weisenden und von einem Kugeloberflächenausschnitt und von einer ebenen Fläche abweichenden Anlagekörperknochenanlagefläche, welche korrespondierend zu einer Knochenoberfläche des Patienten geformt ist. Diese Ausgestaltung ermöglicht es insbesondere, das mindestens eine medizinische Instrument in einer eindeutigen und definierten Weise an eine ganz bestimmt Stelle eines Knochens eines Patienten anzusetzen und gegebenenfalls zu befestigen. Auf diese Weise kann das das mindestens eine medizinische Instrument in einer vor Durchführung eines chirurgischen Eingriffs bestimmten Weise, Position und optional auch Orientierung am Knochen festgelegt werden.

Vorteilhaft ist es, wenn die mindestens eine Befestigungselementaufnahme und die mindestens eine Instrumentenbefestigungselementaufnahme jeweils eine Längsachse definieren, die in der Kupplungsstellung zusammenfallen. Diese Ausgestaltung ermöglicht es, mit einem einzigen Befestigungselement einerseits die mindestens eine Referenzierungseinheit und andererseits das mindestens eine medizinische Instrument am Knochen eines Patienten zu befestigen. Insbesondere kann das Befestigungselement gleichzeitig sowohl die mindestens eine Befestigungselementaufnahme als auch die mindestens eine Instrumentenbefestigungselementaufnahme durchsetzen.

Günstig ist es, wenn die Sägelehre mindestens einen Sägeschlitz für ein Sägeblatt einer Knochensäge umfasst. Insbesondere ist es vorteilhaft, wenn die Sägelehre zwei oder mehr Sägeschlitze aufweist. So muss lediglich eine Sägelehre am Knochen des Patienten befestigt werden, wobei dann zwei oder mehr Sägeschnitte am Knochen vorgenommen werden können.

Günstig ist es, wenn die patientenindividuelle Knochenanlagefläche und/oder die patientenindividuelle Anlagekörperknochenanlagefläche eine Kontur aufweisen, welche mindestens einem Teil einer Knochenoberfläche eines Femurs oder einer Tibia entspricht. So kann das mindestens eine medizinische Instrument in definierter Weise an einem Femur oder einer Tibia eines Patienten angelegt und gegebenenfalls befestigt werden.

Gemäß einer weiteren bevorzugten Ausführungsform der Erfindung kann vorgesehen sein, dass die patientenindividuelle Knochenanlagefläche und/oder die patientenindividuelle Anlagekörperknochenanlagefläche und/oder der Grundkörper und/oder der Anlagekörper durch Gießen, spanende Bearbeitung, selektives Lasersintern, additive Herstellungsverfahren oder 3D-Drucken hergestellt sind. Die angegebenen Herstellungsmöglichkeiten sind rein beispielhaft und nicht limitierend. Es können auch beliebige andere, dem Fachmann geläufige Herstellungsverfahren genutzt werden, um Teile des Systems mit patientenindividuellen Anlageflächen auszubilden.

Vorteilhaft ist es, wenn die patientenindividuelle Knochenanlagefläche Knochenanlageflächenkonturdaten definiert, welche nichtinvasiv bestimmten Knochenkonturdaten des Patienten entsprechen oder im Wesentlichen entsprechen. Beispielsweise können die Knochenkonturdaten des Patienten aus Röntgen-, Magnetresonanz- und/oder Ultraschallaufnahmen stammen. Auf diese Weise kann durch bereitgestellte Knochenkonturdaten die patientenindividuelle Knochenanlagefläche individuell an den Knochen eines Patienten angepasst werden. Die angegebenen Möglichkeiten zur nichtinvasiven Bestimmung von Knochenkonturdaten des Patienten sind rein beispielhaft und nicht limitierend. Denkbar sind auch andere, dem Fachmann geläufige Arten zur nichtinvasiven Bestimmung von Knochenkonturdaten.

Ferner ist es günstig, wenn die patientenindividuelle Anlagekörperknochenanlagefläche Anlagekörperknochenanlageflächenkonturdaten definiert, welche nichtinvasiv bestimmten Knochenkonturdaten des Patienten entsprechen oder im Wesentlichen entsprechen. Insbesondere können auch die nichtinvasiv bestimmten Knochenkonturdaten des Patienten aus Röntgen-, Magnetresonanz- und/oder Ultraschallaufnahmen bestimmter Knochen des Patienten stammen.

Vorzugsweise umfasst das medizinische System mindestens ein Befestigungselement zum Festlegen der Referenzierungseinheit an einem Knochen. Bevorzugt umfasst das medizinische System zwei oder mehr Befestigungselemente der genannten Art. So kann die mindestens eine Referenzierungseinheit und/oder auch das mindestens eine medizinische Instrument in definierter Weise und sicher am Knochen eines Patienten befestigt werden.

Besonders kostengünstig ausbilden lässt sich das medizinische System, wenn das mindestens eine Befestigungselement in Form einer Knochenschraube oder in Form eines Knochenpins oder in Form eines Knochennagels ausgebildet ist. Selbstverständlich können alternativ auch andere, dem Fachmann geläufige und bekannte Befestigungselemente genutzt werden, um die die mindestens eine Referenzierungseinheit und/oder das mindestens eine medizinische Instrument definiert und sicher an einem Knochen festzulegen.

Um eine Position der mindestens einen Referenzierungseinheit im Raum in eindeutiger Weis bestimmen zu können, ist es vorteilhaft, wenn die mindestens eine medizinische Referenzierungseinheit mindestens drei Markerelemente umfasst. Sie kann auch vier, fünf oder noch mehr Markerelemente umfassen. Mehr als drei Markerelemente gestatten es insbesondere, die Genauigkeit einer Positionsbestimmung des mindestens einen Referenzierungselements im Raum zu verbessern. Beispielsweise kann durch zusätzliche Markerelemente eine Positionsbestimmung teilweise redundant durchgeführt werden. Zudem kann beispielsweise ein beschädigtes oder verschmutztes Markerelement bei der Positionsbestimmung der mindestens einen Referenzierungseinheit optional erkannt werden und unberücksichtigt bleiben, ohne eine genaue Positionsbestimmung der mindestens einen Referenzierungseinheit im Raum zu gefährden.

Gemäß einer weiteren bevorzugten Ausführungsform des medizinischen Systems ist es vorteilhaft, wenn es ein chirurgisches Navigationssystem mit mindestens einer Nachweiseinrichtung zum Detektieren der Position der mindestens einen Referenzierungseinheit umfasst. Beispielsweise können in einer Rechen- oder Speichereinheit des Navigationssystems Anordnungen des mindestens einen Markerelements auf dem Trägerelement der mindestens einen Referenzierungseinheit oder aber auch Knochenkonturdaten des Patienten und somit Daten der patientenindividuellen Anlagekörperknochenanlagefläche oder der patientenindividuellen Knochenanlagefläche hinterlegt werden, um so einen Einsatz der mindestens einen Referenzierungseinheit sowie des mindestens einen medizinischen Instruments insbesondere hinsichtlich ihrer Genauigkeit zu optimieren.

Die nachfolgende Beschreibung bevorzugter Ausführungsformen der Erfindung dient im Zusammenhang mit den Zeichnungen der näheren Erläuterung. Es zeigen:
- Figur 1:: eine schematische Gesamtansicht eines Ausführungsbeispiels eines medizinischen Systems, insbesondere umfassend ein chirurgisches Navigationssystem;
- Figur 2:: eine schematische Darstellung eines ersten Ausführungsbeispiels einer Referenzierungseinheit mit einer patientenindividuellen Knochenanlagefläche beim Anlegen an einen Femur;

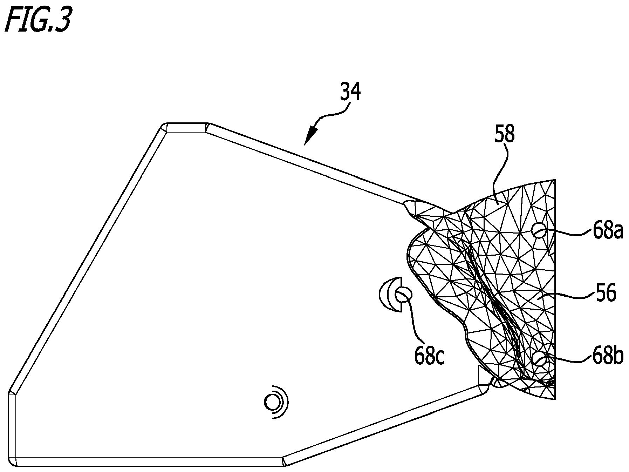

- Figur 3:: eine Ansicht der Referenzierungseinheit aus Figur 2 von unten;
- Figur 4:: eine schematische Darstellung eines ersten Ausführungsbeispiels eines medizinischen Instruments in Form einer Sägelehre vor dem Anlegen und Koppeln mit der an den Knochen angelegten Referenzierungseinheit aus Figur 2;

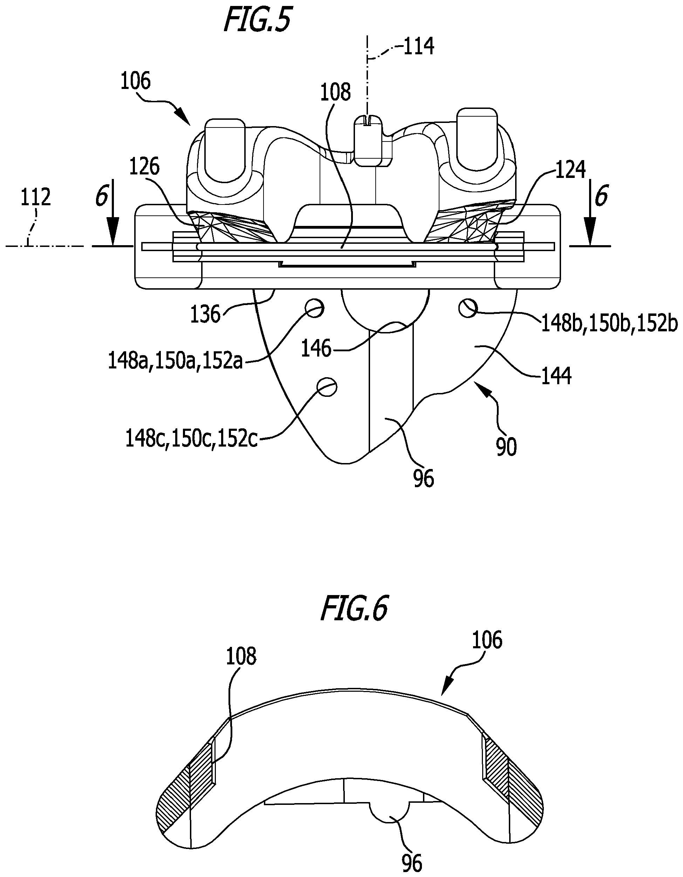

- Figur 5:: eine Ansicht des medizinischen Instruments aus Figur 4 von unten;
- Figur 6:: eine Schnittansicht längs Linie 6 - 6 in Figur 5;
- Figur 7:: eine schematische perspektivische Ansicht der an den Femur angelegten Referenzierungseinheit und des angelegten Instruments aus Figur 4 beim Festlegen derselben mit Knochenpins;
- Figur 8:: eine perspektivische Ansicht der gekoppelten Einheit aus Referenzierungseinheit und medizinischen Instrument nach dem Festlegen am Femur;
- Figur 9:: eine Ansicht der Anordnung aus Figur 8 in Richtung des Pfeils A;
- Figur 10:: eine perspektivische, schematische Ansicht eines weiteren Ausführungsbeispiels einer medizinischen Referenzierungseinheit beim Anlegen und Befestigen an einer Tibia;

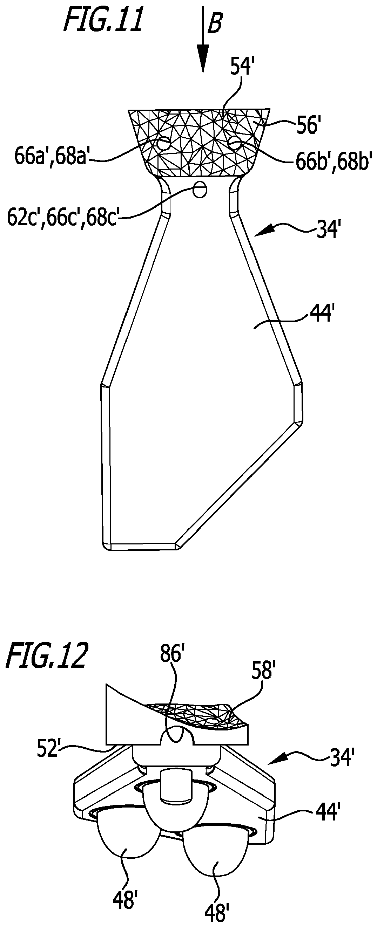

- Figur 11:: eine Ansicht der Referenzierungseinheit aus Figur 10 von unten;
- Figur 12:: eine Ansicht der Referenzierungseinheit in Richtung des Pfeils B in Figur 11;

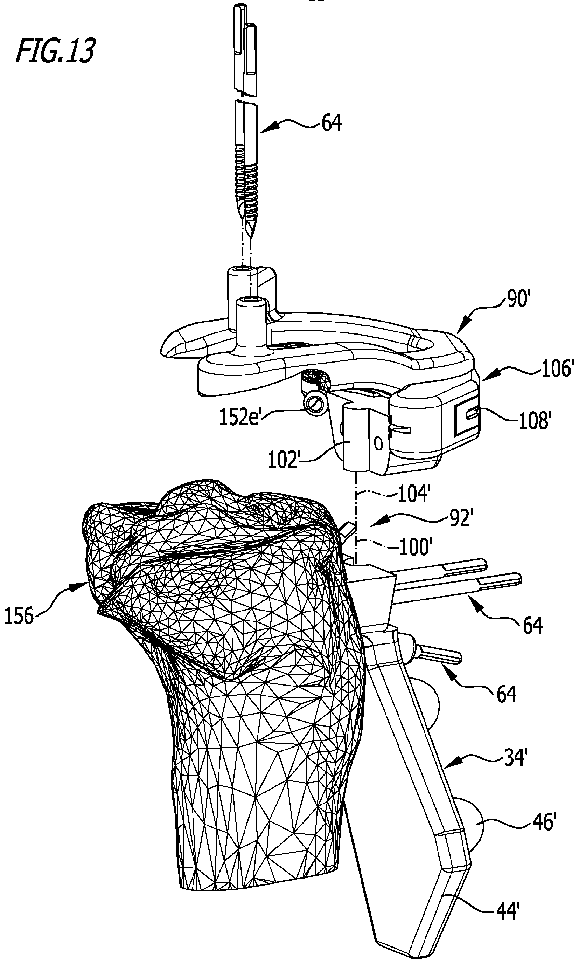

- Figur 13:: eine schematische Gesamtansicht der Referenzierungseinheit aus Figur 10 sowie eines weiteren Ausführungsbeispiels eines medizinischen Instruments vor dem Koppeln derselben und Befestigen an der Tibia;

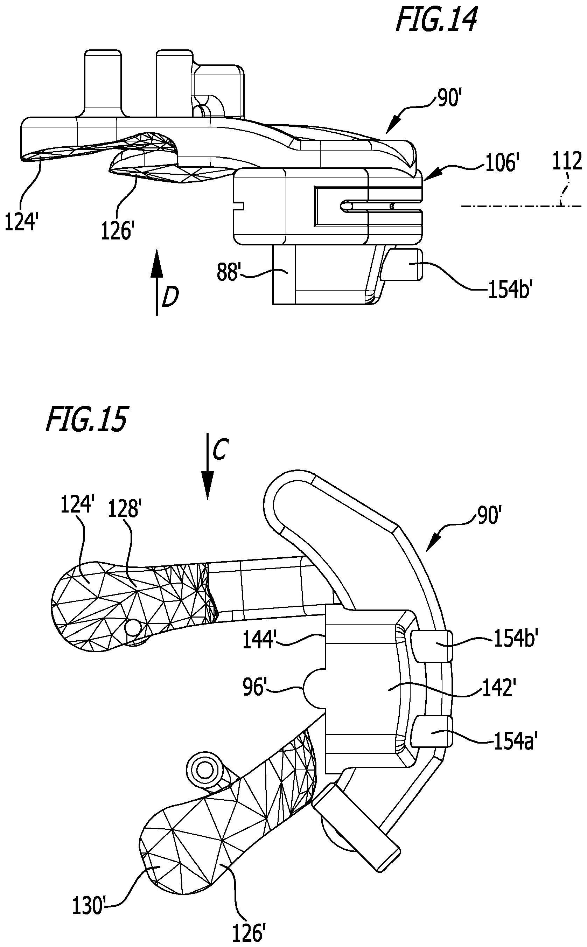

- Figur 14:: eine Seitenansicht des medizinischen Instruments in Richtung des Pfeils C in Figur 15;
- Figur 15:: eine Ansicht des Instruments aus Figur 14 von unten in Richtung des Pfeils D;
- Figur 16:: eine schematische Gesamtansicht der Einheit aus medizinischem Instrument und Referenzierungseinheit aus Figur 13 nach dem Festlegen an der Tibia;
- Figur 17:: eine Ansicht der Anordnung aus Figur 16 von anterior; und
- Figur 18:: eine Ansicht der Anordnung in Figur 16 in Richtung des Pfeils E.

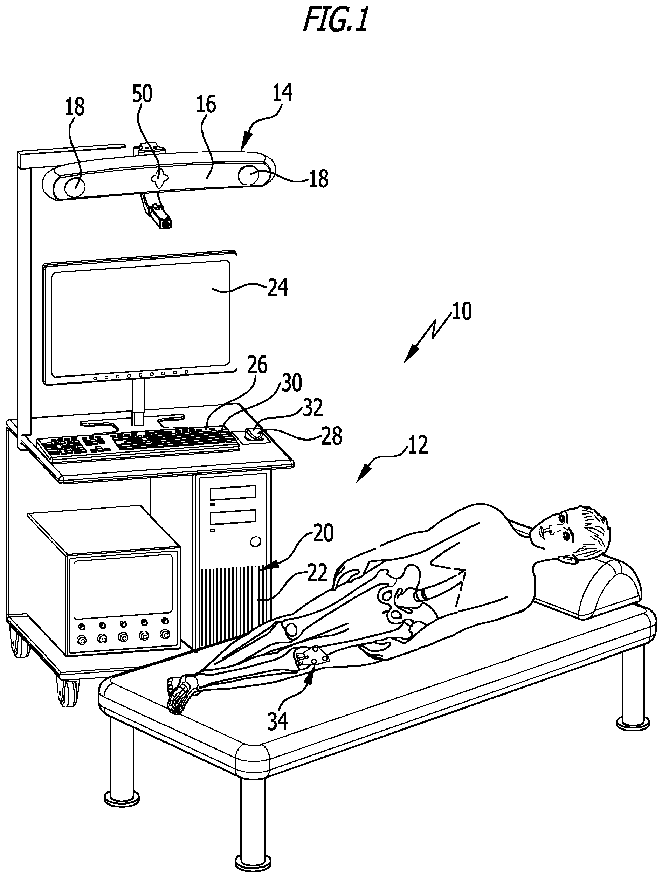

In Figur 1 ist beispielhaft ein medizinisches System 10 dargestellt. Es umfasst insbesondere ein Navigationssystem 12 mit einer Empfangseinheit 14 in Form einer Stereokamera 16 mit zwei Detektoren 18, die vorzugsweise zum Nachweis von elektromagnetischer Strahlung im nahen Infrarot (NIR) ausgebildet sind. Insbesondere kann ihr Nachweisbereich in einem Wellenlängenbereich von etwa 820 nm bis etwa 880 nm liegen.

Das Navigationssystem 12 umfasst ferner eine Datenverarbeitungsanlage 290, welche bei dem in Figur 1 dargestellten Ausführungsbeispiel einen Computer 22, einen Monitor 24 und Eingabegeräte 26 und 28 in Form einer Tastatur 30 und einer Maus 32 umfasst. Der Computer 22 ist mit dem Monitor 24, den Eingabegeräten 26 und 28 sowie mit der Empfangseinheit 14 gekoppelt.

Mit der Datenverarbeitungsanlage 20 können von der Empfangseinheit 14 empfangene Signale verarbeitet werden, um eine Position und/oder Orientierung einer Referenzierungseinheit 34 im Raum zu bestimmen. Auf dem Monitor 24 können insbesondere Lage und Orientierung der Referenzierungseinheit 34 im Operationssaal dargestellt werden.

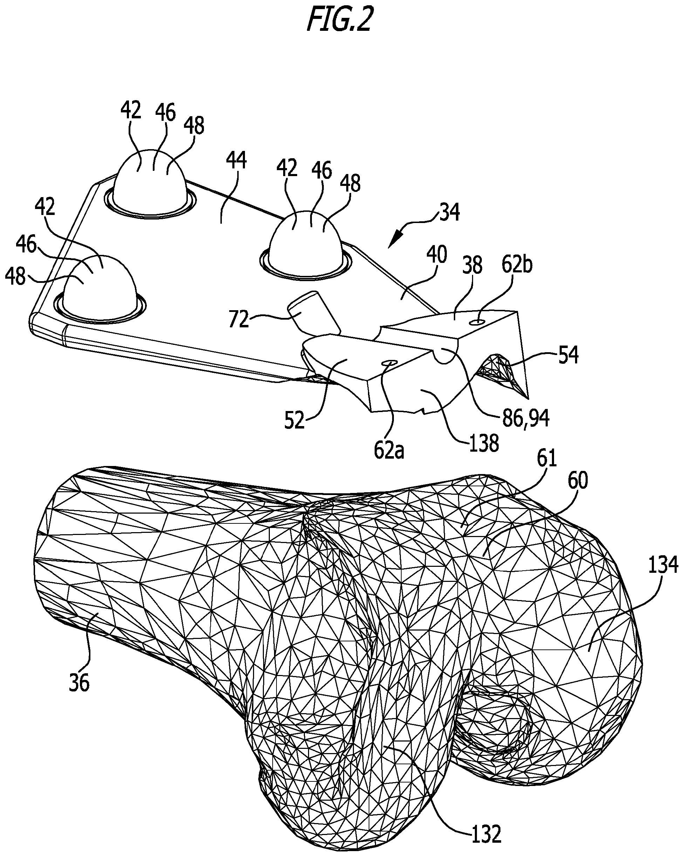

Die Referenzierungseinheit 34 ist schematisch in Figur 2 vor dem Anlegen an einen Femur 36 dargestellt. Sie umfasst einen Grundkörper 38, welcher ein Trägerelement 40 mit Markerelementen 42 trägt. Bei der in Figur 2 dargestellten Referenzierungseinheit 34 ist das Trägerelement 40 in Form einer polygonalen Trägerplatte 44 ausgebildet, auf welcher die Markerelemente 42 angeordnet sind. Diese sind in Form von Halbkugeln 46 ausgebildet, deren Oberflächen 48 elektromagnetische Strahlung oder Ultraschall reflektieren, die beispielsweise von einem Sender 50 des Navigationssystems 12 ausgesandt werden.

Alternativ können die Markerelemente 42 statt in passiver Ausführung wie beschrieben, auch in Form aktiver Markerelemente ausgebildet sein, die beispielsweise elektromagnetische Strahlung oder Ultraschall aussenden.

Statt der Markerelemente 42 können auch definierte Referenzpunkte am Trägerelement 40 vorgesehen werden, beispielsweise in Form definierter Vertiefungen, deren Position am Trägerelement bekannt ist. Diese Referenzpunkte können beispielsweise mit einem navigierten Palpationsinstrument palpiert werden, welches mindestens drei von der Stereokamera 16 detektierbare Markerelemente aufweist. So kann durch Bestimmung der Position des Palpationsinstruments im Raum den Referenzpunkten ihre Position im Raum zugewiesen werden. Ist am Femur 36 eine weitere, in den Figuren nicht dargestellte Referenzierungseinheit mit mindestens einem, vorzugsweise drei chirurgischen Markerelementen festgelegt, ist dann auch die Position der definierten Referenzpunkte relativ zur weiteren Referenzierungseinheit, so dass bei einer Bewegung des Femurs 36 mit dem Trägerelement eine Position der definierten Referenzpunkte durch Bestimmung von Lage und Orientierung der weiteren Referenzierungseinheit zu jeder Zeit über das Navigationssystem bestimmbar ist.

Die Anordnung der Markerelemente 42 relativ zueinander ist vorzugsweise in einem Speicher der Datenverarbeitungsanlage 20 hinterlegt. Die Position der einzelnen Markerelemente 42 im Raum und damit Lage und Orientierung der Referenzierungseinheit 34 insgesamt kann dann in bekannter Weise mit dem Navigationssystem 12 bestimmt werden.

Bei der Referenzierungseinheit 34 aus Figur 2 sind der Grundkörper 38 und das Trägerelement 40 unlösbar miteinander verbunden. Sie können alternativ auch einstückig ausgebildet sein.

Der Grundkörper 38 weist eine ebene Oberseite 52 und eine Unterseite 54 auf. Die Unterseite 54 ist in Form einer patientenindividuellen, vom Grundkörper 38 weg weisenden und von einem Kugeloberflächenausschnitt und von einer ebenen Fläche abweichenden Knochenanlagefläche 56 ausgebildet. Eine Kontur 58 der Knochenanlagefläche 56 entspricht einem Teil 61 einer Knochenoberfläche 60 des Femurs 36.

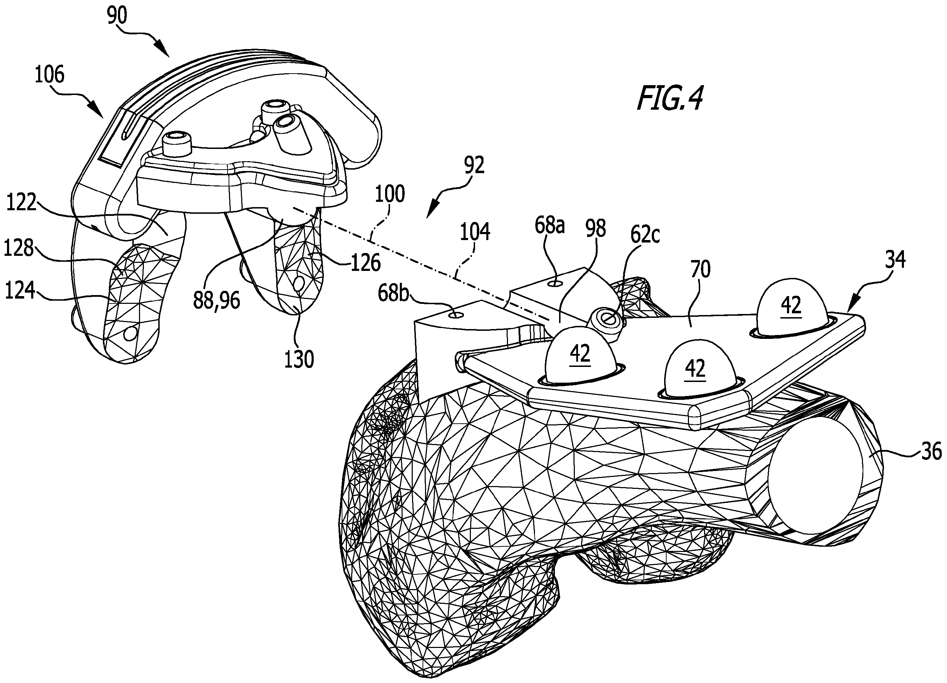

Aufgrund der an die Knochenoberfläche 60 angepassten Kontur 58 der Knochenanlagefläche 56 gibt es genau eine Möglichkeit, den Grundkörper 38 passgenau an den Femur 36 anzulegen. Diese Position der Referenzierungseinheit 34 am Femur 36 ist beispielhaft in Figur 4 dargestellt.

An der Referenzierungseinheit 34 sind ferner Befestigungselementaufnahmen 62a, 62b und 62c vorgesehen. Sie dienen zur Aufnahme jeweils eines Befestigungselements 64 zum Festlegen der Referenzierungseinheit 34 am Femur 36.

Die Befestigungselementaufnahmen 62a, 62b und 62c sind in Form von Durchbrechungen 66a, 66b und 66c ausgebildet, und zwar in Form von Bohrungen 68a, 68b und 68c. Die Bohrungen 68a und 68b verlaufen quer, insbesondere senkrecht zur Oberseite 52 und erstrecken sich durch die Knochenanlagefläche 56 hindurch.

Die Bohrung 68c ist im Bereich der Trägerplatte 44 ausgebildet und durchsetzt das Trägerelement 40. Die Durchbrechung 66c der Trägerplatte 44 ist durch eine von einer Oberseite 70 des Trägerelements 40 abstehende Hülse 72 etwas verlängert, die eine Längsachse definiert, welche windschief zu Längsachsen der Bohrungen 68a und 68b verläuft.

Die Befestigungselemente 64 sind in Form von Knochenpins 74 ausgebildet, die eine Spitze 76 aufweisen, an die sich ein kurzer Außengewindeabschnitt 78 in Form eines Knochengewindes anschließt. An den Außengewindeabschnitt 78 schließt sich ein zylindrischer Schaftabschnitt 80 an, der etwas länger ist als eine halbe Gesamtlänge des Knochenpins 74.

An einem der Spitze 76 gegenüberliegenden Ende des Knochenpins 74 ist ein Mehrkantabschnitt 82 ausgebildet, der bei den in den Figuren dargestellten Knochenpins 74 in Form eines Dreikants ausgebildet ist. Eine Länge des Mehrkantabschnitts 82 parallel zu einer vom Knochenpin 74 definierten Längsachse 84 entspricht in etwa einer Länge des Außengewindeabschnitts 78.

Ein Außendurchmesser des Schaftabschnitts 80 ist an einen Innendurchmesser der Bohrungen 68a, 68b und 68c angepasst, so dass die Knochenpins 74 mit dem Schaftabschnitt 80 die Durchbrechungen 66a, 66b und 66c im Wesentlichen spielfrei durchsetzen können.

Am Grundkörper 38 ist ferner ein erstes Kupplungselement 86 ausgebildet, und zwar korrespondierend zu einem zweiten Kupplungselement 88 eines medizinischen Instruments 90. Die Kupplungselemente 86 und 88 bilden eine Kupplungseinrichtung 92 zum kraft- und/oder formschlüssigen Kuppeln des medizinischen Instruments 90 und der Referenzierungseinheit 34 in einer Kupplungsstellung.

Das erste Kupplungselement 86 ist in Form einer Kupplungsaufnahme 94, das zweite Kupplungselement 88 in Form eines mit dieser zusammenwirkenden, korrespondierenden Kupplungsvorsprungs 96 ausgebildet. Die Kupplungsaufnahme 94 ist in Form einer eine Längsachse 100 definierenden Nut ausgebildet, die einen halbkreisförmigen Querschnitt definiert. Der Kupplungsvorsprung 96 ist in Form einer halbzylinderförmigen Rippe 102 ausgebildet, deren Längsachse 104 parallel zur Längsachse 100 verläuft.

Das Instrument 90 umfasst eine Sägelehre 106 für den Femur 36. Die Sägelehre 106 umfasst zwei Sägeschlitze 108 und 110, die zueinander senkrecht verlaufende Schnittebenen 112 und 114 definieren.

Das Instrument 90 umfasst ferner einen im Wesentlichen U-förmigen Anlagekörper 116, der auf einer Vorderseite 118 einen abstehenden Schlitzkörper 120 trägt, welcher vom Sägeschlitz 110 durchsetzt wird.

Auf einer Rückseite 122 sind im Bereich freier Enden des Anlagekörpers 116 zwei Anlagekörperknochenanlageflächen 124 und 126 ausgebildet, die Konturen 128 und 130 aufweisen, welche Bereichen oder Teilen 132 und 134 der Knochenoberfläche 60 des Femurs 36 entsprechen.

Zwischen den Anlagekörperknochenanlageflächen 124 und 126 ist eine ebene Anschlagfläche 136 ausgebildet, welche in der Kupplungsstellung an einer ebenen Stirnfläche 138 des Grundkörpers 38 anliegt.

Ein zweiter Schlitzkörper 140 ist vom Sägeschlitz 108 durchsetzt und an einem Verbindungsbereich zwischen freien Schenkeln des Anlagekörpers 116 angeordnet. Er trägt einen Kupplungskörper 142 mit einer ebenen Unterseite 144, von der der Kupplungsvorsprung 96 absteht. Der Kupplungskörper 142 ist im Wesentlichen in Form eines Halbrings ausgebildet und begrenzt zusammen mit dem zweiten Schlitzkörper 140 eine im Wesentlichen halbkreisförmige Durchbrechung 146.

In der Kupplungsstellung liegt die Unterseite 144 flächig an der Oberseite 52 des Grundkörpers 38 an.

Der Kupplungskörper 142 weist ferner drei Instrumentenbefestigungselementaufnahmen 148a, 148b und 148c auf, die in Form von Durchbrechungen 150a, 150b und 150c ausgebildet sind, und zwar in Form von Bohrungen 152a, 152b und 152c.

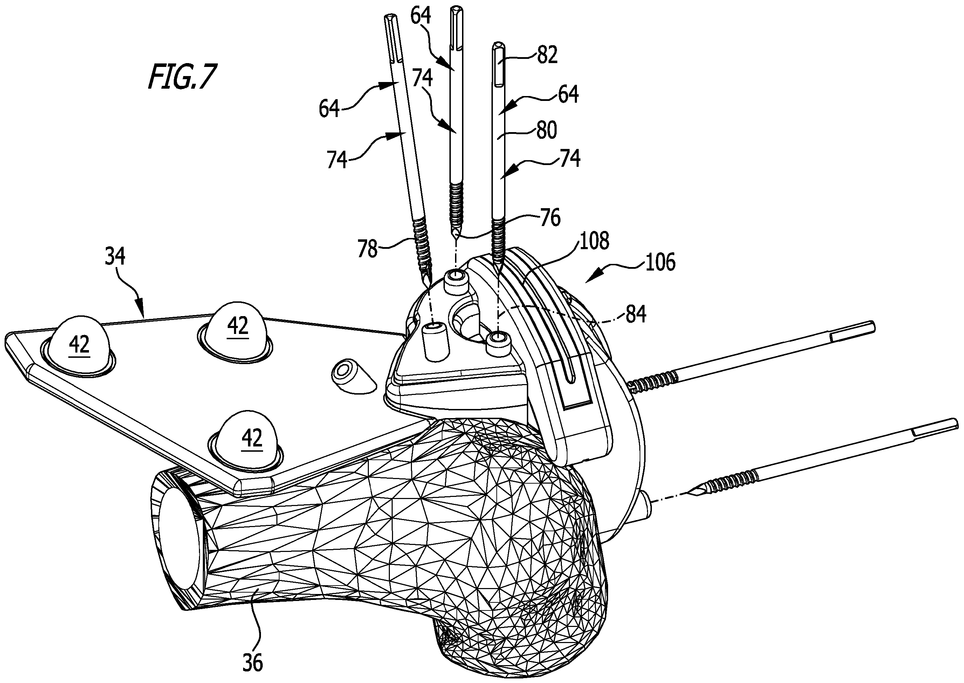

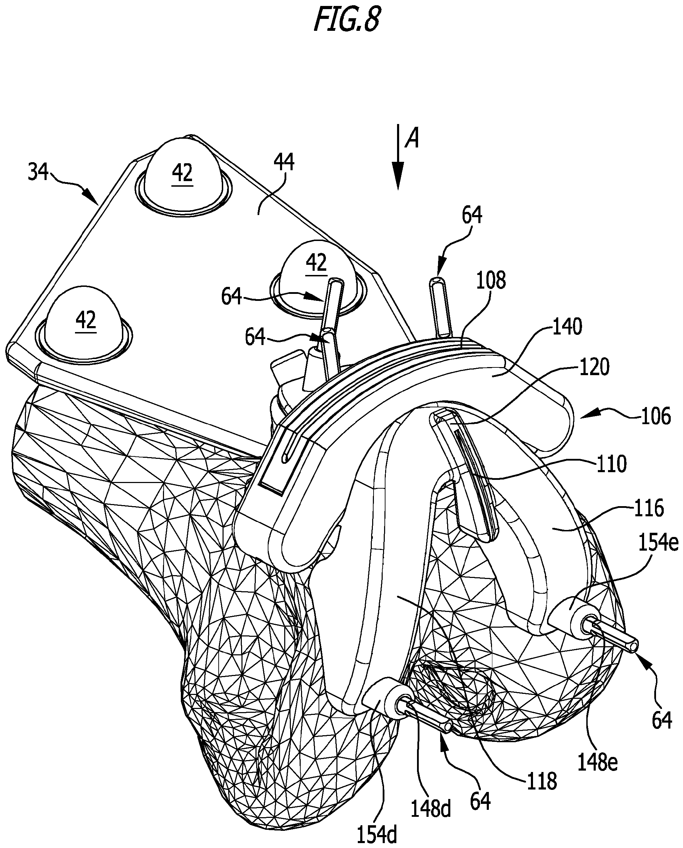

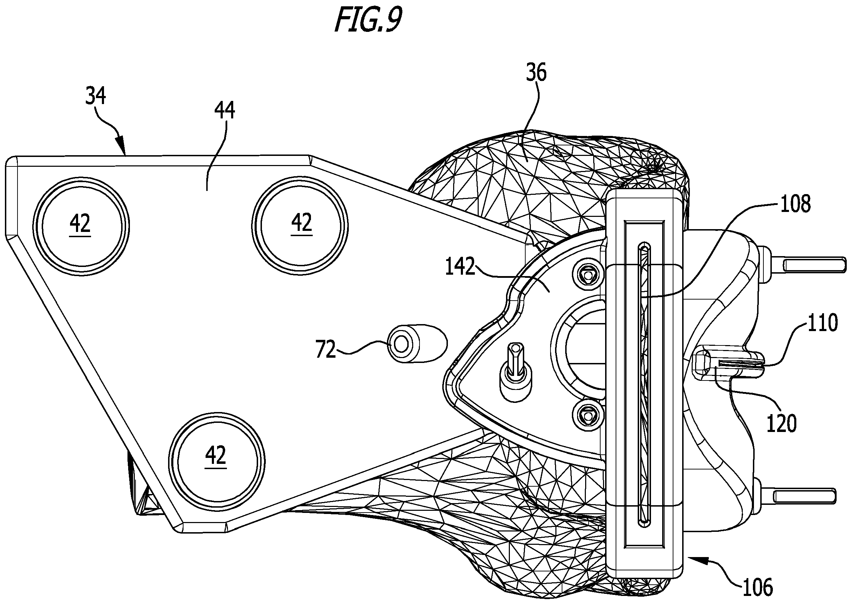

In der Kupplungsstellung fallen Längsachsen der Bohrungen 152a und 152b mit Längsachsen der Bohrungen 68a und 68b zusammen. Dies gestattet es, die Referenzierungseinheit 34 und das Instrument 90 in der Kupplungsstellung mit zwei Befestigungselementen 64 am Femur festzulegen. Dies ist schematisch in den Figuren 7 bis 9 dargestellt.

Um das Instrument 90 zusätzlich am Femur 36 fixieren zu können, sind am Anlagekörper 116 zwei weitere Instrumentenbefestigungselementaufnahmen 148d und 148e vorgesehen, die die Anlagekörperknochenanlageflächen 124 und 126 durchsetzen.

Auch die Instrumentenbefestigungselementaufnahmen 148d und 148e sind durch kurze Hülsen 154d und 154e verlängert. Längsachsen der Instrumentenbefestigungselementaufnahmen 148d und 148e verlaufen im Wesentlichen parallel zueinander und definieren eine Ebene, die senkrecht zu einer von Längsachsen der die Instrumentenbefestigungselementaufnahmen 148a und 148b aufgespannten Ebene verläuft. Die letztgenannte Ebene verläuft zudem parallel zur Schnittebene 112. Die von den Instrumentenbefestigungselementaufnahmen 148d und 148e aufgespannte Ebene verläuft zudem senkrecht zur Schnittebene 114.

Die patientenindividuelle Knochenanlagefläche 56 definiert Knochenanlageflächenkonturdaten, welche nicht invasiv bestimmten Knochenkonturdaten des Patienten entsprechen. Hierzu können insbesondere Knochenkonturdaten des Patienten aus Röntgen-, Magnetresonanz- und/oder Ultraschallaufnahmen genutzt werden.

Auf Basis der so nichtinvasiv bestimmten Knochenkonturdaten des Patienten kann die patientenindividuelle Knochenanlagefläche 56 beziehungsweise der Grundkörper 38 beispielsweise durch Gießen, spanende Bearbeitung oder 3D-Drucken hergestellt werden. Dies bedeutet also, dass für den chirurgischen Eingriff ein Grundkörper 38 ausgebildet wird, welche individuell an eine Kontur des Femurs 36 des Patienten angepasst ist. Aus den nichtinvasiv bestimmten Knochenkonturdaten des Patienten ergibt sich so eine eindeutige Positionierung der Referenzierungseinheit 34 am Femur 36.

In analoger Weise wird auch der Anlagekörper 116 beziehungsweise das gesamte Instrument 90 mit seiner Anlagekörperknochenanlagefläche 124 beziehungsweise 126 ausgebildet. Die patientenindividuelle Anlagekörperknochenanlagefläche definiert Anlagekörperknochenanlageflächenkonturdaten, die nichtinvasiv bestimmten Knochenkonturdaten des Patienten entsprechen. Auch diese Knochenkonturdaten des Patienten können insbesondere aus Röntgen-, Magnetresonanz- und/oder Ultraschallaufnahmen entnommen sein.

Insgesamt ist so eine eindeutige Positionierung des Instruments 90 am Femur 36 möglich. Da zudem die Kupplungseinrichtung 92 eine eindeutige räumliche Beziehung zwischen der Referenzierungseinheit 34 und dem Instrument 90 in der Kupplungsstellung vorgibt, sind somit auch die vom Operateur insbesondere aufgrund der nichtinvasiv bestimmten Bilddaten vorgegebenen Schnittebenen 112 und 114 durch die Sägeschlitze 108 und 110 eindeutig und für den jeweiligen Patienten individuell definiert.

Die Festlegung der Referenzierungseinheit 34 und des medizinischen Instruments 90 zur Vorbereitung des Femurs 36 für die Implantation einer Femurkomponente einer Kniegelenkendoprothese wird nachfolgend erläutert.

Zunächst wird die Referenzierungseinheit 34 mit der Knochenanlagefläche 56 an den Teil 61 der Knochenoberfläche 60 herangeführt, so dass die Referenzierungseinheit 34 in eindeutiger Weise am Femur 36 anliegt. Mit zwei Knochenpins 74 wird die Referenzierungseinheit 34 am Femur 36 vorfixiert. Hierzu werden die Knochenpins 74 durch die Befestigungselementaufnahmen 62a und 62b eingeführt.

Um zusätzlich eine Rotation der Referenzierungseinheit 34 relativ zum Femur 36 zu verhindern, kann optional ein weiterer Knochenpin 74 durch die Befestigungselementaufnahme 62c eingeführt und in den Femur 36 getrieben werden. Dies ist insbesondere empfehlenswert, wenn die Referenzierungseinheit 34 allein, also ohne das Instrument 90 genutzt werden soll, beispielsweise zur Bestimmung einer Lage und Orientierung des Femurs 26 im Raum.

Über die parallel zueinander aus dem Femur 36 vorstehenden und die Durchbrechungen 66a und 66b durchsetzenden Knochenpins 74 kann nun das Instrument 90 an den Femur 36 herangeführt werden. Dazu wird der Kupplungskörper 142 an die Mehrkantabschnitte 82 der bereits gesetzten Knochenpins 74 herangeführt, welche dann durch die Bohrungen 152a und 152b hindurchgeführt werden.

Um ein Verschieben des Instruments 90 relativ zum Femur 36 und zur Referenzierungseinheit 34 zu verhindern, werden zwei weitere Knochenpins 74 durch die Instrumentenbefestigungselementaufnahme 148d und 148e eingeführt und im Femur 36 verankert.

Um mittels des Instruments 90 Sägeschnitte am Femur 36 vorzunehmen, kann optional die Referenzierungseinheit 34 vom Femur 36 entfernt werden, ohne dass das Instrument 90 entfernt werden muss. Hierzu werden die beiden Knochenpins 74, die die Bohrungen 152a und 152b durchsetzen, aus dem Femur 36 entfernt, so dass die Referenzierungseinheit 34 zwischen Femur 36 und Anlagekörper 116 herausgezogen werden kann.

Alternativ ist es auch möglich, das Instrument 90 und die Referenzierungseinheit 34 derart auszubilden, dass das Instrument 90 vom Femur 36 zu entfernbar ist, so dass nur die Referenzierungseinheit 34 am Femur 36 verbleiben kann, um beispielsweise dessen Lage und Orientierung im Raum weiter verfolgen zu können. Hierzu kann insbesondere nach erfolgtem Sägeschnitt das Instrument 90 nach Entfernen der beiden Knochenpins 148e und 148d vom Femur 36 und den beiden Knochenpins 68a und 68b abgezogen werden. Die Referenzierungseinheit 34 verbleibt dann in einer sogenannten "geparkten" Position.

Optional können das Instrument 90 und die Referenzierungseinheit auch zusammen am Femur 36 angelegt werden und nicht in der beschriebenen Reihenfolge.

Alternativ kann auch zuerst das Instrument 90 mit seinen Anlagekörperknochenanlageflächen 124 und 126 an den Femur 36 angelegt und fixiert werden und dann erst das Instrument 90 mit der Referenzierungseinheit 34 gekoppelt werden.

Für eine gute Fixierung des Instruments 90 am Femur 36 können nun die beiden entfernten Knochenpins 74 nochmals eingesetzt werden. Alternativ oder zusätzlich kann auch ein Knochenpin 74 durch die Bohrung 152c eingeführt und im Femur 36 verankert werden, wobei eine Längsachse der Bohrung windschief zu den Längsachsen der Bohrungen 152a und 152b verläuft.

Nachdem mit einer Säge die gewünschten Schnitte am Femur 36 ausgeführt wurden, können alle Knochenpins 74 und auch das medizinische Instrument 90 wieder vom Femur entfernt werden.

Das Instrument 90 kann gegen ein weiteres, in den Figuren nicht dargestelltes Instrument ausgetauscht werden, um weitere Sägeschnitte am Femur 36 vorzunehmen.

An den nun vorbereiteten Femur 36 kann ein in den Figuren nicht dargestelltes Femurteil einer Kniegelenkendoprothese angesetzt und in geeigneter Weise befestigt werden, beispielsweise mittels Knochenschrauben und/oder Knochenzement.

Sowohl die Referenzierungseinheit 34 als auch das medizinische Instrument 90 können vollständig aus einem sterilisierbaren Kunststoff hergestellt sein, beispielswiese aus Polyetheretherketon (PEEK) oder Polyamid 12 (PA 12).

Optional ist es zudem denkbar, den Grundkörper 38 und das Trägerelement 40 miteinander lösbar verbindbar auszubilden. Zu diesem Zweck kann eine in den Figuren nicht dargestellte Kopplungseinrichtung mit ersten und zweiten Kopplungselementen vorgesehen sein, die einerseits am Grundkörper 38 und andererseits am Trägerelement 40 angeordnet beziehungsweise ausgebildet sind. So können dann in ähnlicher Weise wie mit der Kupplungseinrichtung 92, der Grundkörper 38 und das Trägerelement 40 temporär miteinander verbunden werden. Dies ermöglicht es insbesondere, das Trägerelement 40 mit den Markerelementen 42 vom Grundkörper 38 zu trennen. So kann beispielsweise das Trägerelement 40 mit den Markerelementen 42 mehrfach verwendet werden, lediglich der Grundkörper 38 mit seiner patientenindividuellen Knochenanlagefläche 56 muss dann für den chirurgischen Eingriff patientenindividuell hergestellt werden. Auf diese Weise können zusätzlich Kosten reduziert werden.

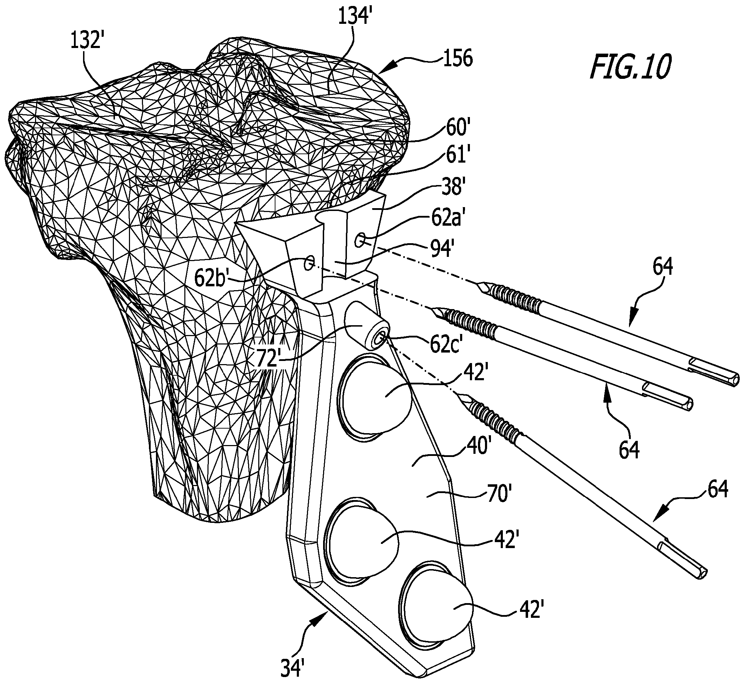

Das medizinische System umfasst optional oder alternativ eine weitere Referenzierungseinheit 34', welche schematisch in Figur 10 vor dem Anlegen an eine Tibia 156 dargestellt ist.

Die Referenzierungseinheit 34' umfasst einen Grundkörper 38', welcher ein Trägerelement 40' mit Markerelementen 42' trägt. Bei der in Figur 10 dargestellten Referenzierungseinheit 34' ist das Trägerelement 40' in Form einer polygonalen Trägerplatte 44' ausgebildet, auf welcher die Markerelemente 42' angeordnet sind. Diese sind in Form von Halbkugeln 46' ausgebildet, deren Oberflächen 48' elektromagnetische Strahlung oder Ultraschall reflektieren.

Alternativ können auch die Markerelemente 42' statt in passiver Ausführung wie bereits oben beschrieben, auch in Form aktiver Markerelemente ausgebildet sein, die beispielsweise elektromagnetische Strahlung oder Ultraschall aussenden.

Die Anordnung der Markerelemente 42' relativ zueinander ist vorzugsweise ebenfalls im Speicher der Datenverarbeitungsanlage 20 hinterlegt. Die Position der einzelnen Markerelemente 42' im Raum und damit Lage und Orientierung der Referenzierungseinheit 34' insgesamt kann dann in bekannter Weise mit dem Navigationssystem 12 bestimmt werden.

Bei der Referenzierungseinheit 34' aus Figur 10 sind der Grundkörper 38' und das Trägerelement 40' unlösbar miteinander verbunden. Sie können alternativ auch einstückig ausgebildet sein.

Der Grundkörper 38 weist eine ebene Oberseite 52' und eine Unterseite 54' auf. Die Unterseite 54' ist in Form einer patientenindividuellen, vom Grundkörper 38' weg weisenden und von einem Kugeloberflächenausschnitt und von einer ebenen Fläche abweichenden Knochenanlagefläche 56' ausgebildet. Eine Kontur 58' der Knochenanlagefläche 56' entspricht einem Teil 61' einer Knochenoberfläche 60' der Tibia 156.

Aufgrund der an die Knochenoberfläche 60' angepassten Kontur 58' der Knochenanlagefläche 56' gibt es genau eine Möglichkeit, den Grundkörper 38' passgenau an die Tibia 156 anzulegen. Diese Position der Referenzierungseinheit 34' an der Tibia 156 ist beispielhaft in Figur 10 dargestellt.

An der Referenzierungseinheit 34' sind ferner Befestigungselementaufnahmen 62a, 62b und 62c vorgesehen. Sie dienen zur Aufnahme jeweils eines Befestigungselements 64 zum Festlegen der Referenzierungseinheit 34 am Femur 36.

Die Befestigungselementaufnahmen 62a', 62b' und 62c' sind in Form von Durchbrechungen 66a', 66b' und 66c' ausgebildet, und zwar in Form von Bohrungen 68a', 68b' und 68c'. Die Bohrungen 68a' und 68b' verlaufen quer, insbesondere senkrecht zur Oberseite 52' und erstrecken sich durch die Knochenanlagefläche 56' hindurch.

Die Bohrung 68c' ist im Bereich der Trägerplatte 44' ausgebildet und durchsetzt das Trägerelement 40'. Die Durchbrechung 66c' der Trägerplatte 44 ist durch eine von einer Oberseite 70' des Trägerelements 40' abstehende Hülse 72' etwas verlängert, die eine Längsachse definiert, welche windschief zu Längsachsen der Bohrungen 68a' und 68b' verläuft.

Zum Festlegen der Referenzierungseinheit 34' an der Tibia 156 werden weitere Befestigungselemente 64 in Form von Knochenpins 74 verwendet.

Ein Außendurchmesser des Schaftabschnitts 80 der Knochenpins 74 ist auch an einen Innendurchmesser der Bohrungen 68a', 68b' und 68c' angepasst, so dass die Knochenpins 74 mit dem Schaftabschnitt 80 die Durchbrechungen 66a', 66b' und 66c' im Wesentlichen spielfrei durchsetzen können.

Am Grundkörper 38' ist ferner ein erstes Kupplungselement 86' ausgebildet, und zwar korrespondierend zu einem zweiten Kupplungselement 88' eines medizinischen Instruments 90'. Die Kupplungselemente 86' und 88' bilden eine Kupplungseinrichtung 92' zum kraft- und/oder formschlüssigen Kuppeln des medizinischen Instruments 90' und der Referenzierungseinheit 34' in einer Kupplungsstellung.

Das erste Kupplungselement 86' ist in Form einer Kupplungsaufnahme 94', das zweite Kupplungselement 88' in Form eines mit dieser zusammenwirkenden, korrespondierenden Kupplungsvorsprungs 96' ausgebildet. Die Kupplungsaufnahme 94' ist in Form einer eine Längsachse 100' definierenden Nut ausgebildet, die einen halbkreisförmigen Querschnitt definiert. Der Kupplungsvorsprung 96' ist in Form einer halbzylinderförmigen Rippe 102' ausgebildet, deren Längsachse 104' parallel zur Längsachse 100' verläuft.

Das Instrument 90' umfasst eine Sägelehre 106' für eine Tibia 156. Die Sägelehre 106' umfasst einen Sägeschlitz 108', welcher eine Schnittebene 112 definiert.

Das Instrument 90' umfasst ferner einen im Wesentlichen U-förmigen Anlagekörper 116', der in einem Verbindungsbereich zwischen freien Schenkeln des Anlagekörpers 116' einen abstehenden Schlitzkörper 120' trägt, welcher vom Sägeschlitz 108' durchsetzt wird.

Auf einer Rückseite 122' sind im Bereich freier Enden des Anlagekörpers 116' zwei Anlagekörperknochenanlageflächen 124' und 126' ausgebildet, die Konturen 128 und 130 aufweisen, welche Bereichen oder Teilen 132' und 134' der Knochenoberfläche 60' der Tibia 156 entsprechen.

Am Schlitzkörper 120' ist ein Kupplungskörper 142' mit einer ebenen Unterseite 144' angeordnet, von der der Kupplungsvorsprung 96' absteht. Der Kupplungskörper 142' ist im Wesentlichen in Form eines Quaders ausgebildet.

In der Kupplungsstellung liegt die Unterseite 144' flächig an der Oberseite 52' des Grundkörpers 38' an.

Der Kupplungskörper 142' weist ferner drei Instrumentenbefestigungselementaufnahmen 148a und 148b auf, die in Form von Durchbrechungen 150a' 150b' ausgebildet sind, und zwar in Form von Bohrungen 152a' und 152b'.

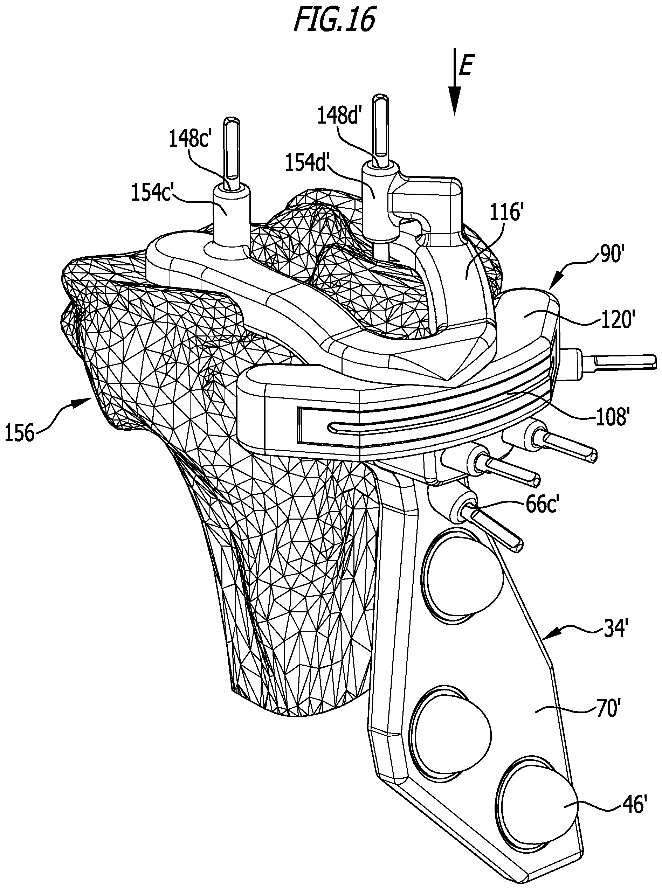

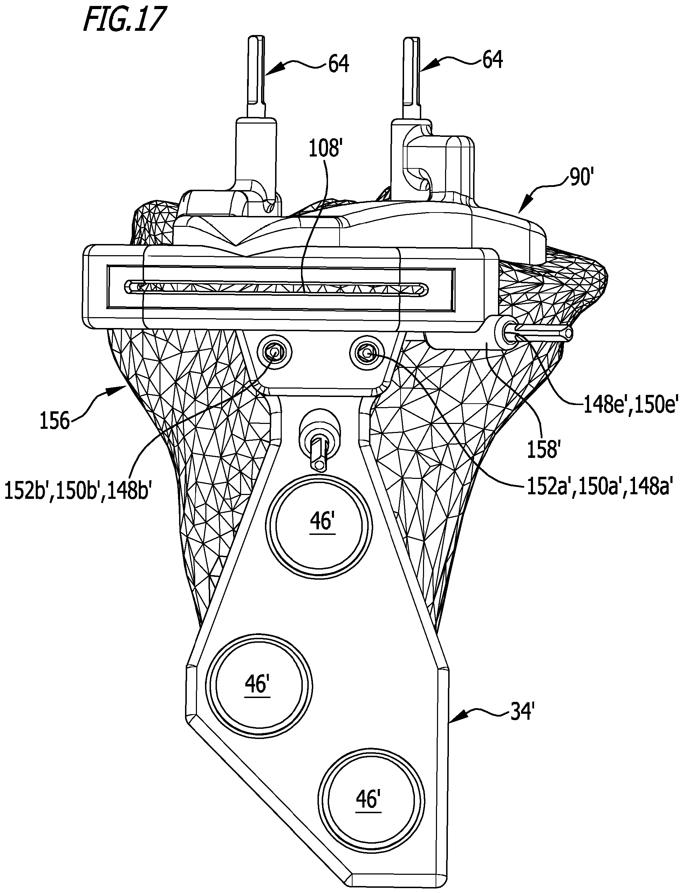

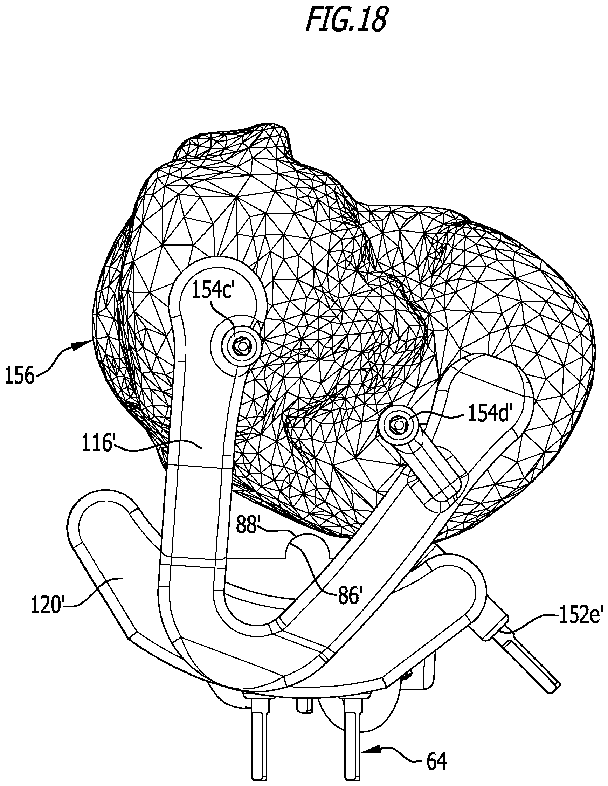

In der Kupplungsstellung fallen Längsachsen der Bohrungen 152a' und 152b' mit Längsachsen der Bohrungen 68a' und 68b' zusammen. Dies gestattet es, die Referenzierungseinheit 34' und das Instrument 90' in der Kupplungsstellung mit zwei Befestigungselementen 64 an der Tibia 156 festzulegen. Dies ist schematisch in den Figuren 16 bis 18 dargestellt.

Um das Instrument 90' zusätzlich an der Tibia 156 fixieren zu können, sind am Anlagekörper 116 zwei weitere Instrumentenbefestigungselementaufnahmen 148c' und 148d' vorgesehen, die die Anlagekörperknochenanlageflächen 124' und 126' durchsetzen.

Am Schlitzkörper 120' ist eine weitere Instrumentenbefestigungselementaufnahme 148e' angeordnet, und zwar in Form einer Durchbrechung 150e' einer Hülse 158. Die Durchbrechung 150e' ist in Form einer Bohrung 152e' der Hülse 158 ausgebildet.

Auch die Instrumentenbefestigungselementaufnahmen 148a', 148b', 148c' und 148d' sind durch kurze Hülsen 154a', 154b', 154c' und 154d' verlängert oder in diesen ausgebildet. Längsachsen der Instrumentenbefestigungselementaufnahmen 148c' und 148d' verlaufen im Wesentlichen parallel zueinander und definieren eine Ebene, die senkrecht zu einer von Längsachsen der die Instrumentenbefestigungselementaufnahmen 148a' und 148b' aufgespannten Ebene verläuft. Die letztgenannte Ebene verläuft zudem parallel zur Schnittebene 112'.

Die patientenindividuelle Knochenanlagefläche 56' definiert Knochenanlageflächenkonturdaten, welche nicht invasiv bestimmten Knochenkonturdaten des Patienten entsprechen. Hierzu können insbesondere Knochenkonturdaten des Patienten aus Röntgen-, Magnetresonanz- und/oder Ultraschallaufnahmen genutzt werden.

Auf Basis der so nichtinvasiv bestimmten Knochenkonturdaten des Patienten kann die patientenindividuelle Knochenanlagefläche 56' beziehungsweise der Grundkörper 38' beispielsweise durch Gießen, spanende Bearbeitung oder 3D-Drucken hergestellt werden. Dies bedeutet also, dass für den chirurgischen Eingriff ein Grundkörper 38' ausgebildet wird, welche individuell an eine Kontur der Tibia 156 des Patienten angepasst ist. Aus den nichtinvasiv bestimmten Knochenkonturdaten des Patienten ergibt sich so eine eindeutige Positionierung der Referenzierungseinheit 34' an der Tibia 156.

In analoger Weise wird auch der Anlagekörper 116' beziehungsweise das gesamte Instrument 90' mit seiner Anlagekörperknochenanlagefläche 124' beziehungsweise 126' ausgebildet. Die patientenindividuelle Anlagekörperknochenanlagefläche definiert Anlagekörperknochenanlageflächenkonturdaten, die nichtinvasiv bestimmten Knochenkonturdaten des Patienten entsprechen. Auch diese Knochenkonturdaten des Patienten können insbesondere aus Röntgen-, Magnetresonanz- und/oder Ultraschallaufnahmen entnommen sein.

Insgesamt ist so eine eindeutige Positionierung des Instruments 90' an der Tibia 156 möglich. Da zudem die Kupplungseinrichtung 92' eine eindeutige räumliche Beziehung zwischen der Referenzierungseinheit 34' und dem Instrument 90' in der Kupplungsstellung vorgibt, ist somit auch die vom Operateur insbesondere aufgrund der nichtinvasiv bestimmten Bilddaten vorgegebene Schnittebene 112' durch den Sägeschlitz 108 eindeutig und für den jeweiligen Patienten individuell definiert.

Die Festlegung der Referenzierungseinheit 34' und des medizinischen Instruments 90' zur Vorbereitung der Tibia 156 für die Implantation einer Femurkomponente einer Kniegelenkendoprothese wird nachfolgend erläutert.

Zunächst wird die Referenzierungseinheit 34' mit der Knochenanlagefläche 56' an den Teil 61' der Knochenoberfläche 60' herangeführt, so dass die Referenzierungseinheit 34' in eindeutiger Weise an der Tibia 156 anliegt. Mit zwei Knochenpins 74 wird die Referenzierungseinheit 34' an der Tibia 156 vorfixiert. Hierzu werden die Knochenpins 74 durch die Befestigungselementaufnahmen 62a' und 62b' eingeführt.

Um zusätzlich eine Rotation der Referenzierungseinheit 34' relativ zur Tibia 156 zu verhindern, kann optional ein weiterer Knochenpin 74 durch die Befestigungselementaufnahme 62c' eingeführt und in die Tibia 156 getrieben werden. Dies ist insbesondere empfehlenswert, wenn die Referenzierungseinheit 34' allein, also ohne das Instrument 90' genutzt werden soll, beispielsweise zur Bestimmung einer Lage und Orientierung der Tibia 156 im Raum.

Über die parallel zueinander aus der Tibia 156 vorstehenden und die Durchbrechungen 66a' und 66b' durchsetzenden Knochenpins 74 kann nun das Instrument 90' an die Tibia 156 herangeführt werden. Dazu wird der Kupplungskörper 142' an die Mehrkantabschnitte 82 der bereits gesetzten Knochenpins 74 herangeführt, welche dann durch die Bohrungen 152a' und 152b' hindurchgeführt werden.

Um ein Verschieben des Instruments 90' relativ zur Tibia 156 und zur Referenzierungseinheit 34' zu verhindern, werden zwei weitere Knochenpins 74 durch die Instrumentenbefestigungselementaufnahme 148c' und 148d'eingeführt und in der Tibia 156 verankert.

Um mittels des Instruments 90' einen Sägeschnitt an der Tibia 156 vorzunehmen, kann optional die Referenzierungseinheit 34' von der Tibia 156 entfernt werden, ohne dass das Instrument 90' entfernt werden muss. Hierzu werden die beiden Knochenpins 74, die die Bohrungen 152a' und 152b' durchsetzen, aus der Tibia 156 entfernt, so dass die Referenzierungseinheit 34' zwischen Tibia 156 und Anlagekörper 116' herausgezogen werden kann.

Alternativ ist es auch möglich, das Instrument 90' und die Referenzierungseinheit 34' derart auszubilden, dass das Instrument 90' von der Tibia 156 entfernbar ist, so dass nur die Referenzierungseinheit 34' an der Tibia 156 verbleiben kann, um beispielsweise deren Lage und Orientierung im Raum weiter verfolgen zu können. Hierzu kann insbesondere nach erfolgtem Sägeschnitt das Instrument 90' nach Entfernen der Knochenpins 148e', 148c' und 148d' von der der Tibia 156 und den beiden Knochenpins 148a' und 148b' abgezogen werden. Die Referenzierungseinheit 34' verbleibt dann in einer sogenannten "geparkten" Position.

Optional können das Instrument 90' und die Referenzierungseinheit auch zusammen an der Tibia 156 angelegt werden und nicht in der beschriebenen Reihenfolge.

Alternativ kann auch zuerst das Instrument 90' mit seinen Anlagekörperknochenanlageflächen 124' und 126' an der Tibia 156 angelegt und fixiert werden und dann erst das Instrument 90' mit der Referenzierungseinheit 34' gekoppelt werden.

Für eine gute Fixierung des Instruments 90' an der Tibia 156 können nun die beiden entfernten Knochenpins 74 nochmals eingesetzt werden. Alternativ oder zusätzlich kann auch ein Knochenpin 74 durch die Bohrung 152e' eingeführt und in der Tibia 156 verankert werden, wobei eine Längsachse der Bohrung 152e' windschief zu den Längsachsen der Bohrungen 152a' und 152b' verläuft.

Nachdem mit einer Säge die gewünschten Schnitte an der Tibia 156 ausgeführt wurden, können alle Knochenpins 74 und auch das medizinische Instrument 90' wieder von der Tibia 156 entfernt werden.

Das Instrument 90' kann gegen ein weiteres, in den Figuren nicht dargestelltes Instrument ausgetauscht werden, um weitere Sägeschnitte an der Tibia 156 vorzunehmen.

An die nun vorbereitete Tibia 156 kann ein in den Figuren nicht dargestelltes Femurteil einer Kniegelenkendoprothese angesetzt und in geeigneter Weise befestigt werden, beispielsweise mittels Knochenschrauben und/oder Knochenzement.

Sowohl die Referenzierungseinheit 34' als auch das medizinische Instrument 90' können vollständig aus einem sterilisierbaren Kunststoff hergestellt sein, beispielswiese aus Polyetheretherketon (PEEK).

Optional ist es zudem denkbar, den Grundkörper 38' und das Trägerelement 40' miteinander lösbar verbindbar auszubilden. Zu diesem Zweck kann eine in den Figuren nicht dargestellte Kopplungseinrichtung mit ersten und zweiten Kopplungselementen vorgesehen sein, die einerseits am Grundkörper 38' und andererseits am Trägerelement 40' angeordnet beziehungsweise ausgebildet sind. So können dann in ähnlicher Weise wie mit der Kupplungseinrichtung 92', der Grundkörper 38' und das Trägerelement 40' temporär miteinander verbunden werden. Dies ermöglicht es insbesondere, das Trägerelement 40' mit den Markerelementen 42' vom Grundkörper 38' zu trennen. So kann beispielsweise das Trägerelement 40' mit den Markerelementen 42' mehrfach verwendet werden, lediglich der Grundkörper 38' mit seiner patientenindividuellen Knochenanlagefläche 56' muss dann für den chirurgischen Eingriff patientenindividuell hergestellt werden. Auf diese Weise können zusätzlich Kosten reduziert werden.

### Bezugszeichenliste

10 medizinisches System
12 Navigationssystem
14 Empfangseinheit
16 Stereokamera
18 Detektor
20 Datenverarbeitungsanlage
22 Computer
24 Monitor
26 Eingabegerät
28 Eingabegerät
30 Tastatur
32 Maus
34, 34' Referenzierungseinheit
36 Femur
38, 38' Grundkörper
40, 40' Trägerelement
42, 42' Markerelement
44, 44' Trägerplatte
46, 46' Halbkugel
48, 48' Oberfläche
50 Sender
52, 52' Oberseite
54, 54' Unterseite
56, 56' Knochenanlagefläche
58, 58' Kontur
60, 60' Knochenoberfläche
61, 61' Teil
62a, 62b, 62c, 62a', 62b', 62c' Befestigungselementaufnahme
64 Befestigungselement
66a, 66b, 66c, 66a', 66b', 66c' Durchbrechung
68a, 68b, 68c, 68a', 68b', 68c' Bohrung
70, 70' Oberseite
72, 72' Hülse
74 Knochenpin
76 Spitze
78 Außengewindeabschnitt
80 Schaftabschnitt
82 Mehrkantabschnitt
84 Längsachse
86, 86' erstes Kopplungselement
88, 88' zweites Kopplungselement
90, 90' medizinisches Instrument
92, 92' Kupplungseinrichtung
94, 94' Kupplungsaufnahme
96, 96' Kupplungsvorsprung
98, 98' Nut
100, 100' Längsachse
102, 102' Rippe
104, 104' Längsachse
106, 106' Sägelehre
108, 108' Sägeschlitz
110 Sägeschlitz
112, 112' Schnittebene
114 Schnittebene
116, 116' Anlagekörper
118, 118' Vorderseite
120, 120' erster Schlitzkörper
122, 122' Rückseite
124, 124' Anlagekörperknochenanlagefläche
126, 126' Anlagekörperknochenanlagefläche
128, 128' Kontur
130, 130' Kontur
132, 132' Teil
134, 134' Teil
136 Anschlagfläche
138 Stirnfläche
140 zweiter Schlitzkörper
142, 142' Kupplungskörper
144, 144' Unterseite
146 Durchbrechung
148a, 148b, 148c, 148d, 148a', 148b', 148c', 148d' Instrumentenbefestigungselementaufnahme
150a, 150b, 150c, 150d, 150a', 150b', 150c', 150d' Durchbrechung
152a, 152b, 152c, 152d, 152a', 152b', 152c', 152' Bohrung
154a, 154b, 154c, 154d, 154a', 154b', 154c', 154d' Hülse
156 Tibia
158 Hülse

## Patentansprüche

1. Medizinisches System (10), insbesondere zum Implantieren einer Kniegelenkendoprothese, umfassend mindestens eine medizinische Referenzierungseinheit (34; 34'), deren Position im Raum mit einem chirurgischen Navigationssystem (12) detektierbar ist, welche Referenzierungseinheit (34; 34') mindestens ein chirurgisches Markerelement (42; 42') umfasst, das auf einem Trägerelement (40; 40') angeordnet oder ausgebildet und mit einer Nachweiseinrichtung (16) des chirurgischen Navigationssystems (12) detektierbar ist, welche mindestens eine Referenzierungseinheit (34; 34') einen Grundkörper (38; 38') umfasst, der das Trägerelement (40; 40') trägt, wobei der Grundkörper(38; 38') mindestens eine patientenindividuelle, vom Grundkörper (38; 38') weg weisende und von einem Kugeloberflächenausschnitt und von einer ebenen Fläche abweichende Knochenanlagefläche (56; 56') aufweist, welche korrespondierend zu einer Knochenoberfläche (60; 60') des Patienten geformt ist, wobei das System mindestens ein medizinisches Instrument (90; 90') umfasst, welches mit der mindestens einen Referenzierungseinheit (34; 34') lösbar verbindbar ist, **dadurch gekennzeichnet, dass** das mindestens eine medizinische Instrument (90; 90') eine Sägelehre (106; 106') für einen Femur (36) und/oder eine Tibia (156) umfasst.

2. Medizinisches System nach Anspruch 1, **dadurch gekennzeichnet, dass** der Grundkörper (38; 38') und das Trägerelement (40; 40') einstückig ausgebildet oder unlösbar miteinander verbunden sind.

3. Medizinisches System nach Anspruch 1, **dadurch gekennzeichnet, dass** der Grundkörper (38; 38') und das Trägerelement (40; 40') lösbar verbindbar ausgebildet sind.

4. Medizinisches System nach Anspruch 1 oder 3, **gekennzeichnet durch** eine Kopplungseinrichtung zum kraft- und/oder formschlüssigen Koppeln des Grundkörpers (38; 38') und des Trägerelements (40; 40') in einer Kopplungsstellung,
wobei insbesondere die Kopplungseinrichtung erste und zweite Kopplungselemente umfasst, die einerseits am Grundkörper (38; 38') und andererseits am Trägerelement (40; 40') angeordnet oder ausgebildet sind und die in der Kopplungsstellung in Eingriff stehen und in einer Trennstellung außer Eingriff stehen,
wobei weiter insbesondere die ersten und zweiten Kopplungselemente mindestens eine Kopplungsaufnahme und einen mit dieser zusammenwirkenden, korrespondierenden Kopplungsvorsprung umfassen.

5. Medizinisches System nach einem der voranstehenden Ansprüche, **dadurch gekennzeichnet, dass** die mindestens eine Referenzierungseinheit (34; 34') mindestens eine Befestigungselementaufnahme (62a, 62b, 62c; 62a', 62b', 62c') für ein Befestigungselement (64) zum Festlegen der Referenzierungseinheit (34; 34') an einem Knochen (36; 156) aufweist.

6. Medizinisches System nach Anspruch 5, **dadurch gekennzeichnet, dass** die mindestens eine Befestigungselementaufnahme (62a, 62b, 62c; 62a', 62b', 62c')
a) am Grundkörper (38; 38') und/oder am Trägerelement (40; 40') angeordnet oder ausgebildet ist
und/oder
b) in Form einer Durchbrechung (66a, 66b, 66c) ausgebildet ist, insbesondere in Form einer Bohrung (68a, 68b, 68c).

7. Medizinisches System nach einem der voranstehenden Ansprüche, **gekennzeichnet durch** eine Kupplungseinrichtung (92; 92') zum kraft- und/oder formschlüssigen Kuppeln des mindestens einen medizinischen Instruments (90; 90') und der mindestens einen Referenzierungseinheit (34; 34') in einer Kupplungsstellung.

8. Medizinisches System nach Anspruch 7, **dadurch gekennzeichnet, dass** die Kupplungseinrichtung (92; 92') erste und zweite Kupplungselemente (86, 88; 86', 88') umfasst, die einerseits an der mindestens einen Referenzierungseinheit (34; 34'), insbesondere am Grundkörper (38; 38'), und andererseits am mindestens einen medizinischen Instrument (90; 90') angeordnet oder ausgebildet sind und die in der Kupplungsstellung in Eingriff stehen und in einer Lösestellung außer Eingriff stehen,
wobei insbesondere die ersten und zweiten Kupplungselemente (86, 88; 86', 88') mindestens eine Kupplungsaufnahme (94; 94') und mindestens einen mit dieser zusammenwirkenden, korrespondierenden Kupplungsvorsprung (96; 96') umfassen.

9. Medizinisches System nach einem der voranstehenden Ansprüche, **dadurch gekennzeichnet, dass** das mindestens eine medizinische Instrument (90; 90') mindestens eine Instrumentenbefestigungselementaufnahme (148a, 148b, 148c, 148d, 148e; 148a', 148b', 148c', 148d' 148e') für ein Befestigungselement (64) zum Festlegen des mindestens einen medizinischen Instruments (90; 90') an einem Knochen (36; 156) aufweist,
wobei insbesondere die mindestens eine Instrumentenbefestigungselementaufnahme (148a, 148b, 148c, 148d, 148e; 148a', 148b', 148c', 148d' 148e') in Form einer Durchbrechung (150a, 150b, 150c, 150d, 150e; 150a', 150b', 150c', 150d' 150e')ausgebildet ist, insbesondere in Form einer Bohrung (152a, 152b, 152c, 152d, 152e; 152a', 152b', 152c', 152d' 152e').

10. Medizinisches System nach einem der voranstehenden Ansprüche, **dadurch gekennzeichnet, dass**
a) das mindestens eine medizinische Instrument (90; 90') einen Anlagekörper (116; 116') umfasst mit mindestens einer patientenindividuellen, vom Anlagekörper weg weisenden und von einem Kugeloberflächenausschnitt und von einer ebenen Fläche abweichenden Anlagekörperknochenanlagefläche (124, 126; 124', 126'), welche korrespondierend zu einer Knochenoberfläche des Patienten geformt ist
und/oder
b) die Sägelehre (106; 106') mindestens einen Sägeschlitz (108, 110; 108', 110') für ein Sägeblatt einer Knochensäge umfasst.

11. Medizinisches System nach Anspruch 9 oder 10, **dadurch gekennzeichnet, dass** die mindestens eine Befestigungselementaufnahme (62a, 62b; 62a', 62b')und die mindestens eine Instrumentenbefestigungselementaufnahme (148a, 148b; 148a', 148b') jeweils eine Längsachse definieren, die in der Kupplungsstellung zusammenfallen.

12. Medizinisches System nach Anspruch 10 oder 11, **dadurch gekennzeichnet, dass** die patientenindividuelle Knochenanlagefläche (56; 56') und/ oder die patientenindividuelle Anlagekörperknochenanlagefläche (124, 126; 124', 126') eine Kontur (58, 128, 130; 58', 128', 130') aufweisen, welche mindestens einem Teil (61; 61') einer Knochenoberfläche (60; 60') eines Femurs (26) oder einer Tibia (156) entspricht.

13. Medizinisches System nach einem der voranstehenden Ansprüche, **dadurch gekennzeichnet,**
a) **dass** die patientenindividuelle Knochenanlagefläche (56; 56') und/ oder die patientenindividuelle Anlagekörperknochenanlagefläche (124, 126; 124', 126') und/oder der Grundkörper (38; 38') und/ oder der Anlagekörper (116; 116') durch Gießen, spanende Bearbeitung oder 3D-Drucken hergestellt sind
und/oder
b) **dass** die patientenindividuelle Knochenanlagefläche (56; 56') Knochenanlageflächenkonturdaten definiert, welche nichtinvasiv bestimmten Knochenkonturdaten des Patienten entsprechen oder im Wesentlichen entsprechen, insbesondere Knochenkonturdaten des Patienten aus Röntgen-, Magnetresonanz- und/oder Ultraschallaufnahmen.

14. Medizinisches System nach einem der Ansprüche 10 bis 13, **dadurch gekennzeichnet, dass** die patientenindividuelle Anlagekörperknochenanlagefläche (124, 126; 124', 126') Anlagekörperknochenanlageflächenkonturdaten definiert, welche nichtinvasiv bestimmten Knochenkonturdaten des Patienten entsprechen oder im Wesentlichen entsprechen, insbesondere Knochenkonturdaten des Patienten aus Röntgen-, Magnetresonanz- und/oder Ultraschallaufnahmen.

15. Medizinisches System nach einem der voranstehenden Ansprüche, **dadurch gekennzeichnet,**
a) **dass** das System mindestens ein Befestigungselement (64) umfasst zum Festlegen der Referenzierungseinheit (34; 34') an einem Knochen,
wobei insbesondere das mindestens eine Befestigungselement (64) in Form einer Knochenschraube oder in Form eines Knochenpins (74) oder in Form eines Knochennagels ausgebildet ist
und/oder
b) **dass** die mindestens eine medizinische Referenzierungseinheit (34; 34') mindestens drei Markerelemente (42; 42') umfasst
und/oder
c) **dass** das System ein chirurgisches Navigationssystem (12) umfasst mit mindestens einer Nachweiseinrichtung (16) zum Detektieren der Position der mindestens einen Referenzierungseinheit (34; 34').

## Claims

1. Medical system (10), in particular for implanting a knee joint endoprosthesis, comprising at least one medical referencing unit (34; 34') whose position in space is detectable using a surgical navigation system (12), which referencing unit (34; 34') comprises at least one surgical marker element (42; 42') that is arranged or formed on a carrier element (40; 40') and is detectable using a detection device (16) of the surgical navigation system (12), which at least one referencing unit (34; 34') comprises a base body (38; 38') that carries the carrier element (40; 40'), wherein the base body (38; 38') comprises at least one patient-specific bone contact face (56; 56') facing away from the base body (38; 38') and deviating from being a sector of a surface of a sphere and from being a planar surface, said at least one bone contact face (56; 56') being formed in a manner corresponding to a bone surface (60; 60') of the patient, wherein the system comprises at least one medical instrument (90; 90') which is releasably connectable to the at least one referencing unit (34; 34'), **characterized in that** the at least one medical instrument (90; 90') comprises a saw template (106; 106') for a femur (36) and/or a tibia (156).

2. Medical system in accordance with claim 1, **characterized in that** the base body (38; 38') and the carrier element (40; 40') are of one-piece configuration or are non-releasably connected together.

3. Medical system in accordance with claim 1, **characterized in that** the base body (38; 38') and the carrier element (40; 40') are configured for releasable connection to each other.

4. Medical system in accordance with claim 1 or 3, **characterized by** a coupling device for force-locking and/or form-locking coupling of the base body (38; 38') and the carrier element (40; 40') in a coupling position, wherein, in particular, the coupling device comprises first and second coupling elements which are arranged or formed on the base body (38; 38') on the one hand and the carrier element (40; 40') on the other hand and are in engagement when in the coupling position and are out of engagement when in an uncoupling position,
wherein further, in particular, the first and second coupling elements comprise at least one coupling receptacle and a corresponding coupling projection cooperating therewith.

5. Medical system in accordance with any one of the preceding claims, **characterized in that** the at least one referencing unit (34; 34') comprises at least one fastening element receptacle (62a, 62b, 62c; 62a', 62b', 62c') for a fastening element (64) for fixing the referencing unit (34; 34') to a bone (36; 156).

6. Medical system in accordance with claim 5, **characterized in that** the at least one fastening element receptacle (62a, 62b, 62c; 62a', 62b', 62c')
a) is arranged or formed on the base body (38; 38') and/or on the carrier element (40; 40')
and/or
b) is configured in the form of an opening (66a, 66b, 66c), in particular in the form of a bore (68a, 68b, 68c).

7. Medical system in accordance with any one of the preceding claims, **characterized by** a coupling device (92; 92') for force-locking and/or form-locking coupling of the at least one medical instrument (90; 90') and the at least one referencing unit (34; 34') in a coupling position.

8. Medical system in accordance with claim 7, **characterized in that** the coupling device (92; 92') comprises first and second coupling elements (86, 88; 86', 88') which are arranged or formed on the at least one referencing unit (34; 34') on the one hand, in particular on the base body (38; 38'), and on the at least one medical instrument (90; 90') on the other hand and are in engagement when in the coupling position and are out of engagement when in an uncoupling position,
wherein, in particular,
the first and second coupling elements (86, 88; 86', 88') comprise at least one coupling receptacle (94; 94') and at least one corresponding coupling receptacle (96; 96') cooperating therewith.

9. Medical system in accordance with any one of the preceding claims, **characterized in that** the at least one medical instrument (90; 90') comprises at least one instrument fastening element receptacle (148a, 148b, 148c, 148d, 148e; 148a', 148b', 148c', 148d', 148e') for a fastening element (64) for fixing the at least one medical instrument (90; 90') to a bone (36; 156),
wherein, in particular, the at least one instrument fastening element receptacle (148a, 148b, 148c, 148d, 148e; 148a', 148b', 148c', 148d', 148e') is configured in the form of an opening (150a, 150b, 150c, 150d, 150e; 150a', 150b', 150c', 150d', 150e'), in particular in the form of a bore (152a, 152b, 152c, 152d, 152e; 152a', 152b', 152c', 152d', 152e').

10. Medical system in accordance with any one of the preceding claims, **characterized in that**
a) the at least one medical instrument (90; 90') comprises a contact body (116; 116') comprising at least one patient-specific contact body bone contact face (124, 126; 124', 126') facing away from the contact body and deviating from being a sector of a surface of a sphere and from being a planar surface, said at least one patient-specific contact body bone contact face (124, 126; 124', 126') being formed in a manner corresponding to a bone surface of the patient
and/or
b) the saw template (106; 106') comprises at least one saw slot (108, 110; 108') for a saw blade of a bone saw.

11. Medical system in accordance with claim 9 or 10, **characterized in that** the at least one fastening element receptacle (62a, 62b; 62a', 62b') and the at least one instrument fastening element receptacle (148a, 148b; 148a', 148b') each define a longitudinal axis, said longitudinal axes coinciding in the coupling position.

12. Medical system in accordance with claim 10 or 11, **characterized in that** the patient-specific bone contact face (56; 56') and/or the patient-specific contact body bone contact face (124, 126; 124', 126') have a contour (58, 128, 130; 58', 128', 130') which corresponds to at least a part (61; 61') of a bone surface (60; 60') of a femur (26) or of a tibia (156).

13. Medical system in accordance with any one of the preceding claims, **characterized**
a) **in that** the patient-specific bone contact face (56; 56') and/or the patient-specific contact body bone contact face (124, 126; 124', 126') and/or the base body (38; 38') and/or the contact body (116; 116') are manufactured by casting, moulding, chip-producing machining methods or 3-D printing
and/or
b) **in that** the patient-specific bone contact face (56; 56') defines bone contact face contour data which correspond or substantially correspond to non-invasively determined bone contour data of the patient, in particular bone contour data of the patient from X-ray, magnetic resonance and/or ultrasound images.

14. Medical system in accordance with any one of claims 10 to 13, **characterized in that** the patient-specific contact body bone contact face (124, 126; 124', 126') defines contact body bone contact face contour data that correspond or substantially correspond to non-invasively determined bone contour data of the patient, in particular bone contour data of the patient from X-ray, magnetic resonance and/or ultrasound images.

15. Medical system in accordance with any one of the preceding claims, **characterized**
a) **in that** the system comprises at least one fastening element (64) for fixing the referencing unit (34; 34') to a bone,
wherein, in particular, the least one fastening element (64) is configured in the form of a bone screw or in the form of a bone pin (74) or in the form of a bone nail,
and/or
b) **in that** the least one referencing unit (34; 34') comprises at least three marker elements (42; 42')
and/or
c) **in that** the system comprises a surgical navigation system (12) comprising at least one detection device (16) for detecting the position of the at least one referencing unit (34; 34').

## Revendications

1. Système médical (10), en particulier pour l'implantation d'une endoprothèse de l'articulation du genou, comprenant au moins une unité de référencement médicale (34 ; 34'), dont la position dans l'espace est détectable avec un système de navigation chirurgical (12), laquelle unité de référencement (34 ; 34') comprend au moins un élément de marquage chirurgical (42 ; 42'), qui est agencé ou formé sur un élément de support (40 ; 40') et qui est détectable avec une installation de mise en évidence (16) du système de navigation chirurgical (12), laquelle au moins une unité de référencement (34 ; 34') comprend un corps de base (38 ; 38'), qui porte l'élément de support (40 ; 40'), où le corps de base (38 ; 38') présente au moins une surface d'appui d'os (56 ; 56') individuelle pour le patient, tournée vers l'opposé du corps de base (38 ; 38') et différente d'un secteur de surface sphérique et d'une surface plane, qui est formée d'une manière correspondant à une surface d'os (60 ; 60') du patient, où le système comprend au moins un instrument médical (90 ; 90'), qui peut être relié de manière amovible avec la au moins une unité de référencement (34 ; 34'), **caractérisé en ce que** le au moins un instrument médical (90 ; 90') comprend un gabarit de scie (106 ; 106') pour un fémur (36) et/ou un tibia (156).

2. Système médical selon la revendication 1, **caractérisé en ce que** le corps de base (38 ; 38') et l'élément de support (40 ; 40') sont formés d'une pièce ou sont reliés l'un à l'autre de manière non amovible.

3. Système médical selon la revendication 1, **caractérisé en ce que** le corps de base (38 ; 38') et l'élément de support (40 ; 40') sont formés de manière à pouvoir être reliés de manière amovible.

4. Système médical selon la revendication 1 ou 3, **caractérisé par** une installation d'accouplement pour l'accouplement par assemblage de force et/ou de forme du corps de base (38 ; 38') et de l'élément de support (40 ; 40') dans une position d'accouplement,
où en particulier l'installation d'accouplement comprend des premier et second éléments d'accouplement, qui sont agencés ou formés d'une part sur le corps de base (38 ; 38') et d'autre part sur l'élément de support (40 ; 40') et qui sont en prise dans la position d'accouplement et hors de prise dans une position de séparation,
où en outre en particulier les premier et second éléments d'accouplement comprennent au moins un logement d'accouplement et une protubérance d'accouplement correspondante, qui coopère avec celui-ci.

5. Système médical selon l'une des revendications précédentes, **caractérisé en ce que** la au moins une unité de référencement (34 ; 34') présente au moins un logement d'élément de fixation (62a, 62b, 62c ; 62a', 62b', 62c') pour un élément de fixation (64) pour la fixation de l'unité de référencement (34 ; 34') sur un os (36 ; 156).

6. Système médical selon la revendication 5, **caractérisé en ce que** le au moins un logement d'élément de fixation (62a, 62b, 62c ; 62a', 62b', 62c')
a) est agencé ou formé sur le corps de base (38 ; 38') et/ou sur l'élément de support (40 ; 40')
et/ou
b) est formé sous forme d'un perçage (66a, 66b, 66c), en particulier sous forme d'un alésage (68a, 68b, 68c).

7. Système médical selon l'une des revendications précédentes, **caractérisé par** une installation de couplage (92 ; 92') pour le couplage par assemblage de force et/ou de forme du au moins un instrument médical (90 ; 90') et de la au moins une unité de référencement (34 ; 34') dans une position de couplage.

8. Système médical selon la revendication 7, **caractérisé en ce que** l'installation de couplage (92 ; 92') comprend des premier et second éléments de couplage (86, 88 ; 86', 88'), qui sont agencés ou formés d'une part sur la au moins une unité de référencement (34 ; 34'), en particulier sur le corps de base (38 ; 38'), et d'autre part sur au moins un instrument médical (90 ; 90') et qui sont en prise dans la position de couplage et sont hors de prise dans une position de séparation,
où en particulier les premier et second éléments de couplage (86, 88 ; 86', 88') comprennent au moins un logement de couplage (94 ; 94') et au moins une protubérance de couplage (96 ; 96') correspondante, qui coopère avec celui-ci.

9. Système médical selon l'une des revendications précédentes, **caractérisé en ce que** le au moins un instrument médical (90 ; 90') présente au moins un logement d'élément de fixation d'instrument (148a, 148b, 148c, 148d, 148e ; 148a', 148b', 148c', 148d', 148e') pour un élément de fixation (64) pour la fixation du au moins un instrument médical (90 ; 90') sur un os (36 ; 156),
où en particulier le au moins un logement d'élément de fixation d'instrument (148a, 148b, 148c, 148d, 148e ; 148a', 148b', 148c', 148d', 148e') est formé sous forme d'un perçage (150a, 150b, 150c, 150d, 150e ; 150a', 150b', 150c', 150d', 150e'), en particulier sous forme d'un alésage (152a, 152b, 152c, 152d, 152e ; 152a', 152b', 152c', 152d', 152e').

10. Système médical selon l'une des revendications précédentes, **caractérisé en ce que**
a) le au moins un instrument médical (90 ; 90') comprend un corps d'appui (116 ; 116') avec au moins une surface d'appui d'os de corps d'appui (124, 126 ; 124', 126') individuelle pour le patient, tournée vers l'opposé du corps d'appui et différente d'un secteur de surface sphérique et d'une surface plane, qui est formée d'une manière correspondant à une surface d'os du patient
et/ou
b) le gabarit de scie (106 ; 106') comprend au moins une rainure de scie (108, 110 ; 108', 110') pour une lame de scie d'une scie à os.

11. Système médical selon la revendication 9 ou 10, **caractérisé en ce que** le au moins un logement d'élément de fixation (62a, 62b ; 62a', 62b') et le au moins un logement d'élément de fixation d'instrument (148a, 148b ; 148a', 148b') définissent l'un et l'autre des axes longitudinaux qui coïncident dans la position de couplage.

12. Système médical selon la revendication 10 ou 11, **caractérisé en ce que** la surface d'appui d'os (56 ; 56') individuelle pour le patient et/ou la surface d'appui d'os de corps d'appui (124, 126 ; 124', 126') individuelle pour le patient présentent un contour (58, 128, 130 ; 58', 128', 130') qui correspond au moins à une partie (61 ; 61') d'une surface d'os (60 ; 60') d'un fémur (26) ou d'un tibia (156).

13. Système médical selon l'une des revendications précédentes, **caractérisé**
a) **en ce que** la surface d'appui d'os (56 ; 56') individuelle pour le patient et/ou la surface d'appui d'os de corps d'appui (124, 126 ; 124', 126') individuelle pour le patient et/ou le corps de base (38 ; 38') et/ou le corps d'appui (116 ; 116') sont fabriqués par coulée, traitement par enlèvement de copeaux ou impression 3 D
et/ou
b) **en ce que** la surface d'appui d'os (56 ; 56') individuelle pour le patient définit des données de contour de surface d'appui d'os qui correspondent ou correspondent sensiblement à des données de contour d'os du patient déterminées de manière non invasive, en particulier des données de contour d'os du patient provenant d'enregistrements aux rayons X, de résonance magnétique et/ou aux ultrasons.

14. Système médical selon l'une des revendications 10 à 13, **caractérisé en ce que** la surface d'appui d'os de corps d'appui (124, 126 ; 124', 126') individuelle pour le patient définit des données de contour de surface d'appui d'os de corps d'appui qui correspondent ou correspondent sensiblement à des données de contour d'os du patient déterminées de manière non invasive, en particulier des données de contour d'os du patient provenant d'enregistrements aux rayons X, de résonance magnétique et/ou aux ultrasons.

15. Système médical selon l'une des revendications précédentes, **caractérisé**
a) **en ce que** le système comprend au moins un élément de fixation (64) pour la fixation de l'unité de référencement (34 ; 34') sur un os, où en particulier le au moins un élément de fixation (64) est formé sous forme d'une vis à os ou sous forme d'une broche à os (74) ou sous forme d'un clou à os
et/ou
b) **en ce que** la au moins une unité de référencement médicale (34 ; 34') comprend au moins trois éléments de marquage (42 ; 42')
et/ou
c) **en ce que** le système comprend un système de navigation chirurgical (12) avec au moins une installation de mise en évidence (16) pour la détection de la position de la au moins une unité de référencement (34 ; 34').
